# EUROPEAN PATENT SPECIFICATION

(11) **EP 0 694 068 B1**
(45) Date of publication and mention of the grant of the patent: **12.02.2003**
(21) Application number: 94912200.6
(22) Date of filing: 08.03.1994
(51) Int. Cl.: C12N 15/12, C12N 15/67, C12N 9/90, C12N 15/10, C12N 5/10, C12P 21/08, C07K 7/06, C12Q 1/68, G01N 33/68, A61K 38/00

(54) **GENETIC SUPPRESSOR ELEMENTS ASSOCIATED WITH SENSITIVITY TO CHEMOTHERAPEUTIC DRUGS**
MIT CHEMOTHERAPEUTISCHE ZUSAMMENSETZUNGENASSOZIIERTEN GENETISCHEN SUPPRESSOR-ELEMENTEN
ELEMENTS SUPPRESSEURS GENETIQUES ASSOCIES A UNE SENSIBILITE AUX MEDICAMENTS CHIMIOTHERAPIQUES

(30) Priority: 09.03.1993 US 33086
(43) Date of publication of application: 31.01.1996
(73) Proprietor: THE BOARD OF TRUSTEES OF THE UNIVERSITY OF ILLINOIS, Urbana, Illinois 61801 (US)
(72) Inventor: RONINSON, Igor B., Wilmette, IL 60091 (US); GUDKOV, Andrei, Apartment 1S, Chicago, IL 60637 (US)
(74) Representative: Bizley, Richard Edward
(86) International application number: US9402519
(87) International publication number: WO94020618

(56) References cited:
- WO-A-92/07071
- JOURNAL OF BIOLOGICAL CHEMISTRY. vol. 265, no. 6 , 25 February 1990 , BALTIMORE US pages 3278 - 3283 KOSIK, K.ET AL.; 'The primary structure and analysis of the squid kinesin heavy chain.'
- NUCLEIC ACIDS RESEARCH. vol. 20, no. 4 , 25 February 1992 , ARLINGTON, VIRGINIA US pages 711 - 717 HOLZMAYER TA;PESTOV DG;RONINSON IB; 'Isolation of dominant negative mutants and inhibitory antisense RNA sequences by expression selection of random DNA fragments.' cited in the application
- PROCEEDINGS OF THE NATIONAL ACADEMY OF SCIENCES OF USA. vol. 90, no. 8 , 15 April 1993 , WASHINGTON US pages 3231 - 3235 GUDKOV AV;ZELNICK CR;KAZAROV AR;THIMMAPAYA R;SUTTLE DP;BECK WT;RONINSON IB; 'Isolation of genetic suppressor elements, inducing resistance to topoisomerase II-interactive cytotoxic drugs, from human topoisomerase II cDNA.' cited in the application

## Description

### BACKGROUND OF THE INVENTION

### 1. Field Of The Invention

The invention relates to genetic factors associated with sensitivity to chemotherapeutic drugs. More particularly, the invention relates to methods for identifying such factors as well as to uses for such factors.

### 2. Summary Of The Related Art

A broad variety of chemotherapeutic agents are used in the treatment of human cancer. For example the textbook *CANCER: Principles & Practice of Oncology,* 2d Edition, (De Vita *et al.,* Eds.), J.B. Lippincott Company, Philadelphia, PA (1985) discloses as major antineoplastic agents the plant alkaloids vincristine, vinblastine, vindesine, and VM-26; the antibiotics actinomycin-D, doxorubicin, daunorubicin, mithramycin, mitomycin C and bleomycin; the antimetabolites methotrexate, 5-fluorouracil, 5-fluorodeoxyuridine, 6-mercaptopurine, 6-thioguanine, cytosine arabinoside, 5-aza-cytidine and hydroxyurea; the alkylating agents cyclophosphamide, melphalan, busulfan, CCNU, MeCCNU, BCNU, streptozotocin, chlorambucil, bis-diaminedichloro-platinum, azetidinylbenzoquinone; and the miscellaneous agents dacarbazine, mAMSA and mitoxantrone.

These and other chemotherapeutic agents such as etoposide and amsacrine have proven to be very useful in the treatment of cancer. Unfortunately, some tumor cells become resistant to specific chemotherapeutic agents, in some instances even to multiple chemotherapeutic agents. Such drug resistance or multiple drug resistance can theoretically arise from either the presence of genetic factors that confer resistance to the drugs, or from the absence of genetic factors that confer sensitivity to the drugs. The former type of factors have been identified, and include the multiple drug resistance gene *mdr-1* (see Chen *et al*., 1986, *Cell* 47: 381-389). However, the latter type of factor remains largely unknown, perhaps in part because such absence of factors would tend to be a recessive trait.

Identification of genes associated with sensitivity to chemotherapeutic agents is desirable, because the discovery of such genes can lead to both diagnostic and therapeutic approaches for cancer cells and for drug resistant cancer cells, as well as to improvements in gene therapy and rational drug design. Recently, some developments have been made in the difficult area of isolating recessive genetic elements, including one involved in cytotoxic drug sensitivity. Roninson et al, US Patent No 5,217,889, issued June 8, 1993, teaches a generalized method for obtaining genetic suppressor elements (GSEs), which are dominant negative factors that confer the recessive-type phenotype for the gene to which the particular GSE corresponds. (See also Holzmayer et al, 1992, Nucleic Acids Res 20:711-717). Gudkov et al (1993, Proc Natl Acad Sci USA 90: 3231-3235) teaches isolation of GSEs inducing resistance to topoisomerase II-interactive drugs from topoisomerase II cDNA. However, there remains a need for identifying yet unknown genes or genetic elements associated with sensitivity to chemotherapeutic agents, a task made more difficult by the unavailability of a cloned gene as starting material for preparing GSEs. Preferably such genes or genetic elements will be involved in a common pathway that is implicated in sensitivity to more than one chemotherapeutic agent. Most preferably, such genes or genetic elements will be identified by direct selection of GSEs causing loss of the drug sensitivity phenotype.

### BRIEF SUMMARY OF THE INVENTION

The invention relates to genetic suppressor elements (GSEs) that confer upon cells resistance to chemotherapeutic drugs. These GSEs are random fragments derived from genes associated with sensitivity to chemotherapeutic drugs, although the nature of such genes can be quite surprising. Thus, in a first aspect the invention provides a GSE having a nucleotide sequence as shown in Figure 9 (SEQ ID NO.15), Figure 10 (SEQ ID NO.16) or Figure 11 (SEQ ID NO.17) or its complement.

In a second aspect the invention provides a synthetic peptide having an amino acid sequence corresponding to the amino acid sequence encoded by a GSE of the invention. In a third aspect the invention provides a synthetic oligonucleotide having a nucleotide sequence of an antisense RNA encoded by a GSE of the invention.

In a fourth aspect the invention provides the use of a GSE of the invention for conferring etoposide resistance on a eukaryotic cell.

In a fifth aspect the invention provides the use of a GSE of the invention in a method of isolating a gene associated with sensitivity to a chemotherapeutic drug comprising the step of screening a cDNA library with the GSE.

In a sixth aspect the invention provides the use of a GSE of the invention for diagnosing chemotherapeutic drug resistance in a tumour sample, the use comprising the step of assaying the tumour sample for expression of a gene that hybridises to the GSE wherein drug resistant tumour samples have reduced or absent expression of said gene.

In a seventh aspect the invention provides a mammalian cell that expresses a GSE of the invention.

In a eighth aspect the invention provides an antibody reactive to a protein or polypeptide encoded by a GSE of the invention.

In a ninth aspect the invention provides the use of a GSE of the invention for the manufacture of a medicament for the treatment of cancer.

In a tenth aspect the invention provides a GSE of the invention for use as a pharmaceutical.

In a eleventh aspect the invention provides the use of a GSE of the invention to identify genes associated with chemotherapeutic drug resistance or senescence.

In a twelfth aspect the invention provides the use of a GSE of the invention for the manufacture of a medicament for enhancing chemotherapy.

### BRIEF DESCRIPTION OF THE DRAWINGS

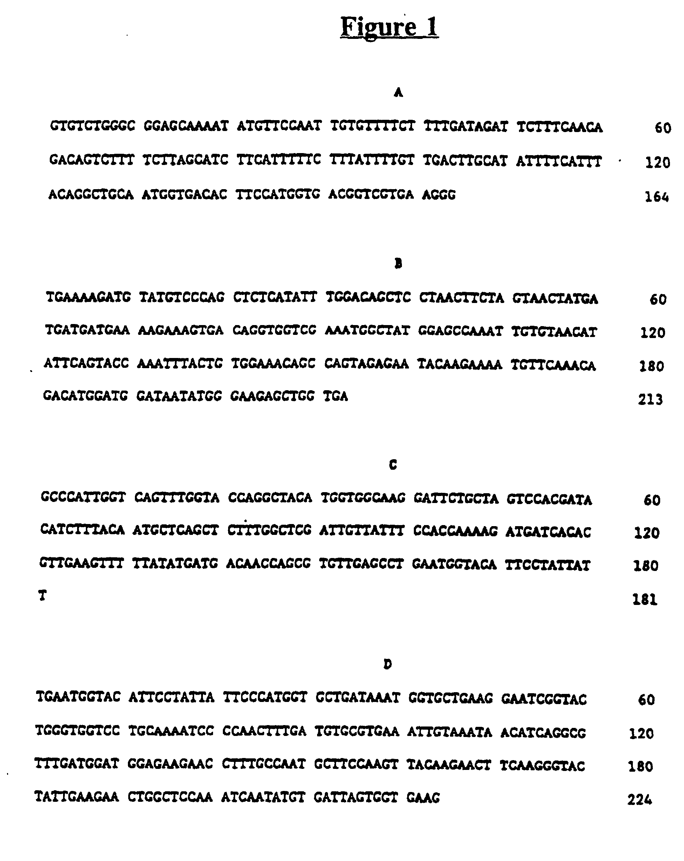

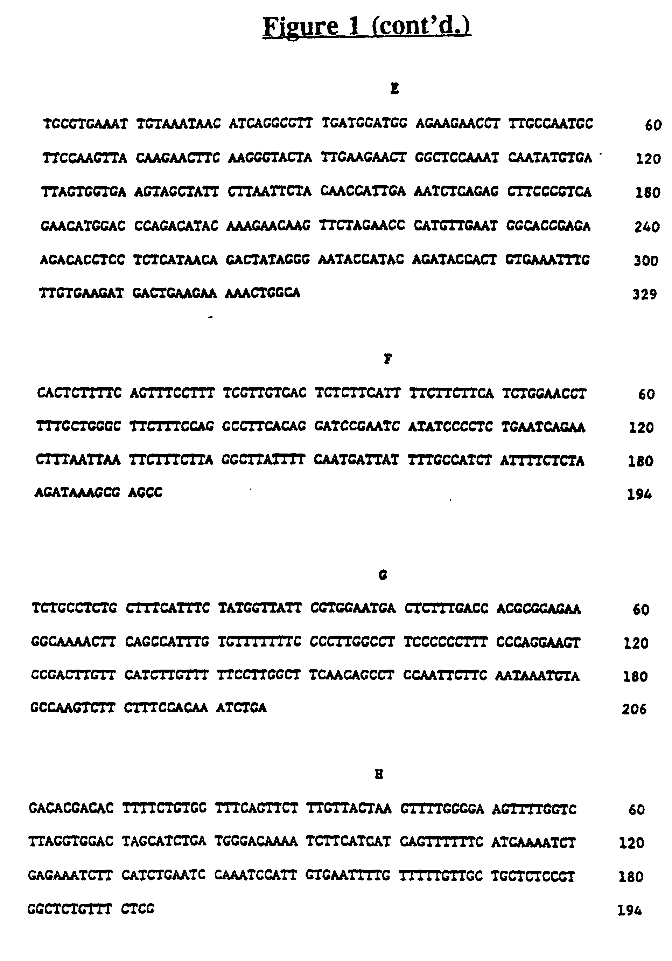

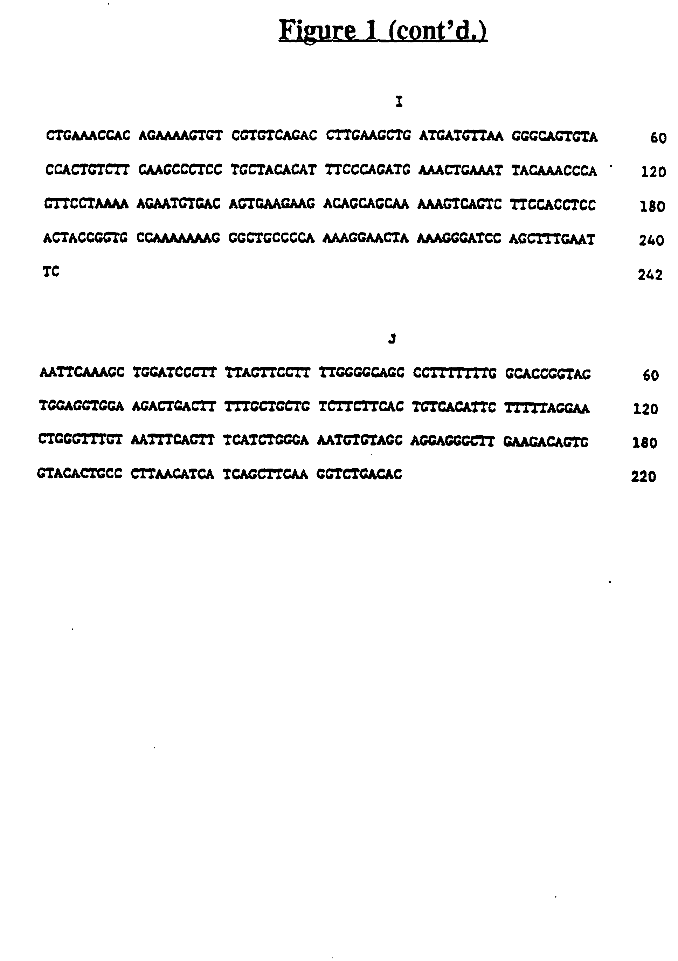

Figure 1 shows the nucleotide sequences of twelve GSEs not within the scope of the present invention that confer etoposide resistance upon cells and that were derived from topoisomerase II DNA, using a single gene random fragment expression library, as described in Example 1. The GSEs shown are (A) clone 2V [SEQ ID NO 1], (B) clone Σ11 [SEQ ID NO 2], (C) clone 6 [SEQ ID NO 3], (D) clone 5 [SEQ ID NO 4], (E) clone Σ28 [SEQ ID NO 5], (F) clone Σ2 [SEQ ID NO 6], (G) clone Σ20 [SEQ ID NO 7], (H) clone 39 [SEQ ID NO 8], (I) clone 12S [SEQ ID NO 9], (J) clone Σ8 [SEQ ID NO 10], (K) clone ΣVPs2 [SEQ ID NO 11], and (L) clone ΣVM [SEQ ID NO 12].

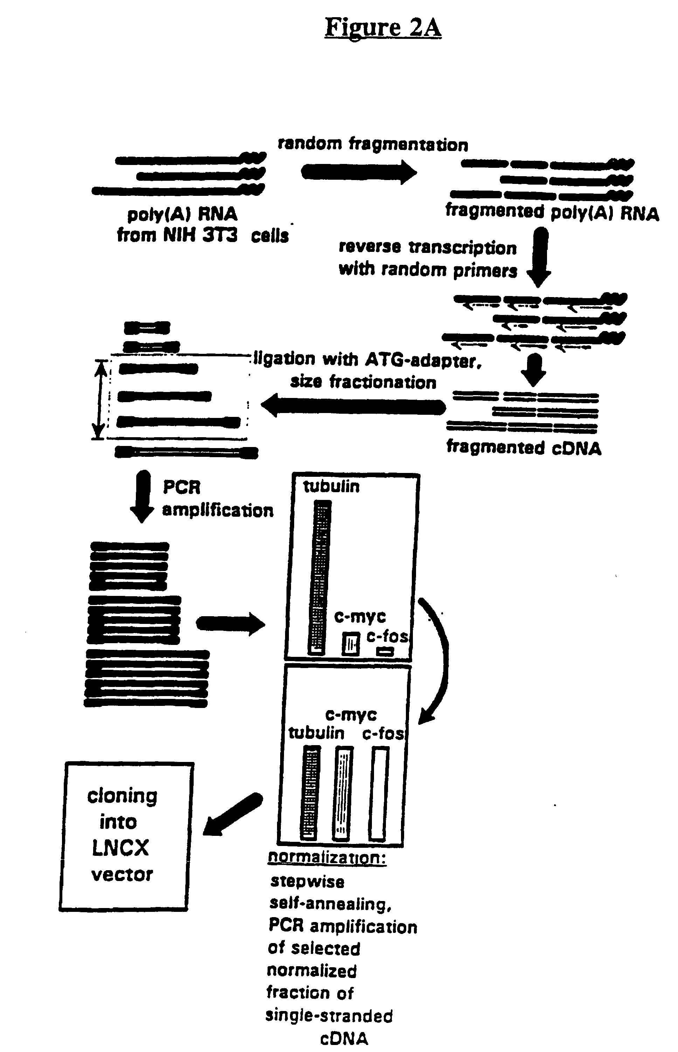

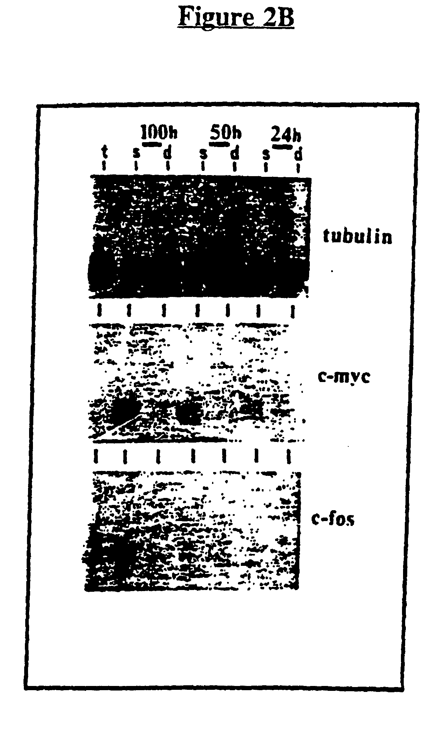

Figure 2 shows a scheme for construction of RFEL from NIH 3T3 cDNA. Panel A shows the overall construction scheme. Panel B shows normalization of the cDNA fragments. In this panel, t represents total unfractionated cDNA, s and d represent the single-stranded and double stranded fractions separated by hydroxyapatite, time points indicate the period of reannealing and tubulin, c-myc, and c-fos indicate the probes used in Southern hybridisation with the total, single-stranded and double-stranded fractions.

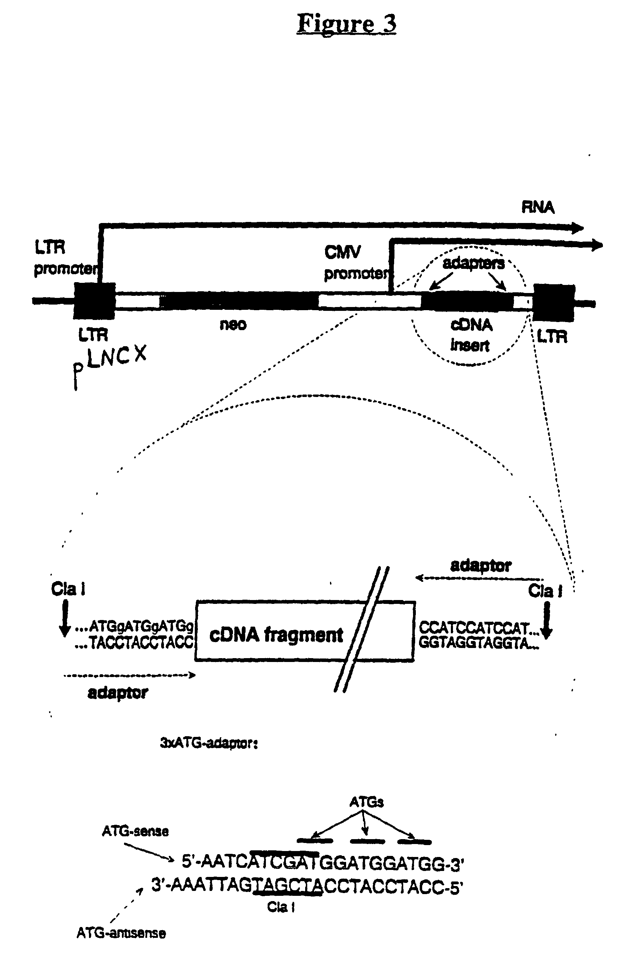

Figure 3 shows the structure of the LNCX vector and the adaptor used in cDNA cloning. The nucleotide sequences are shown for the ATG-sense [SEQ ID NO 13] and ATG-antisense [SEQ ID NO 14] strands of the adaptor.

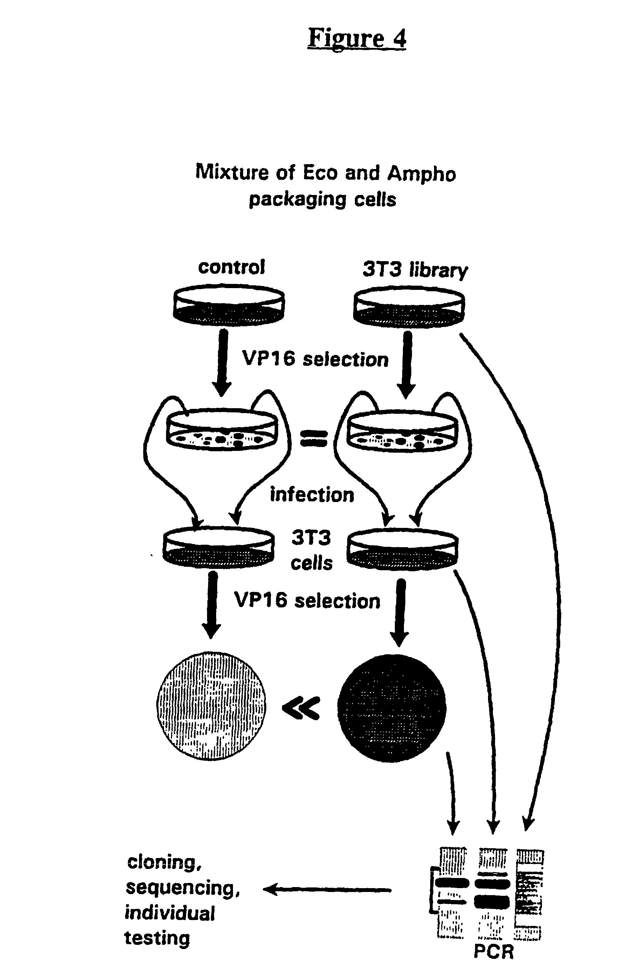

Figure 4 shows the overall scheme for selecting cell lines containing chemotherapeutic drug resistance-conferring GSEs and rescuing the GSEs from these cells.

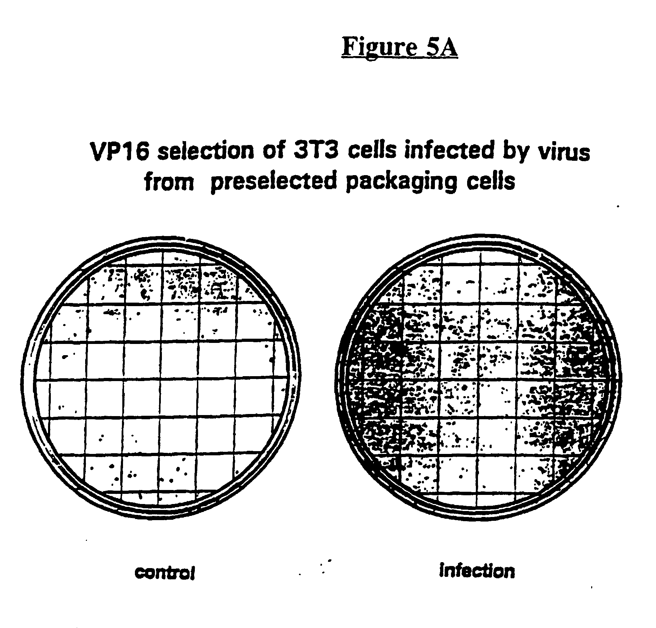

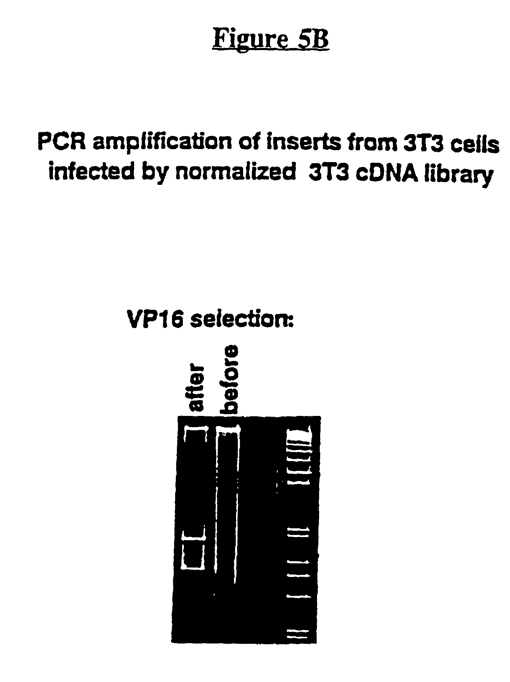

Figure 5 shows the etoposide resistance conferred by preselected virus (Panel A) and PCR analysis of the selected and unselected populations (Panel B).

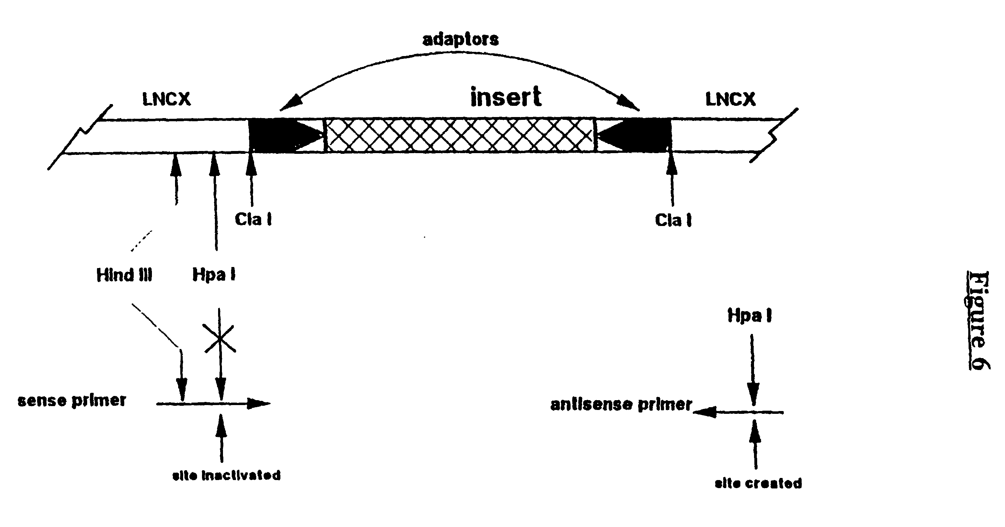

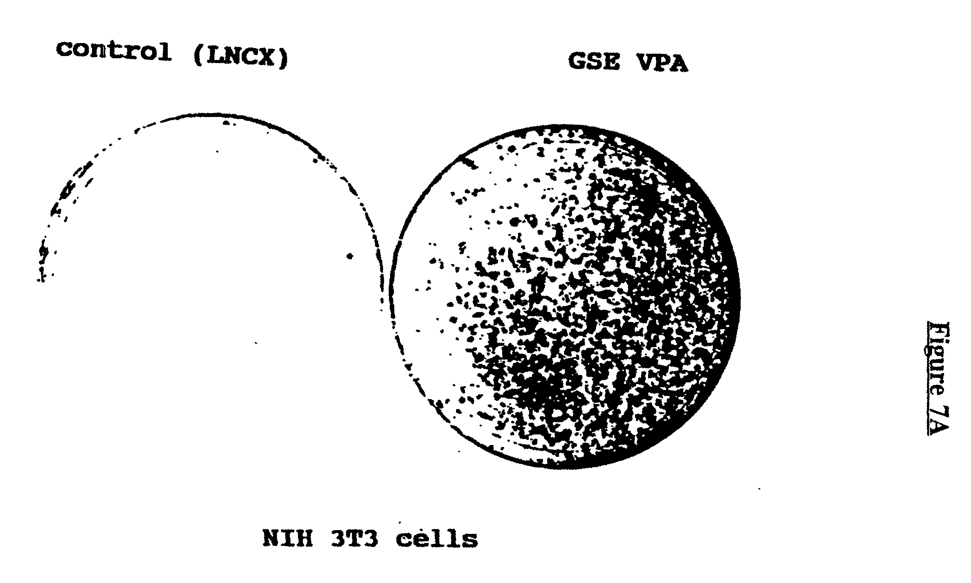

Figure 6 shows a scheme for recloning individual PCR-amplified fragments from etoposide resistant selected cells into the LNCX vector, as described in Example 4.

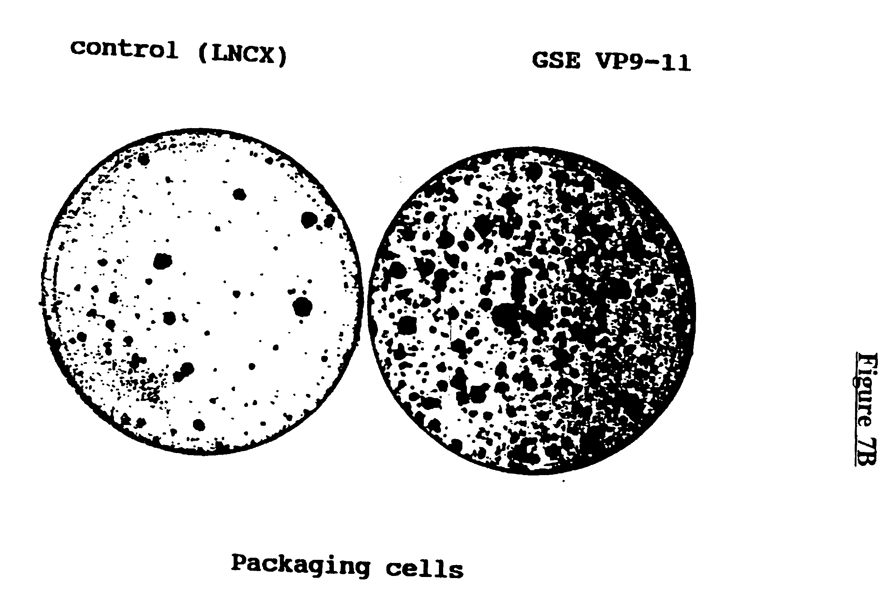

Figure 7 demonstrates resistance to 350 ng/ml etoposide, conferred upon the cells by the GSEs VPA (Panel A) and VP9-11 (Panel B).

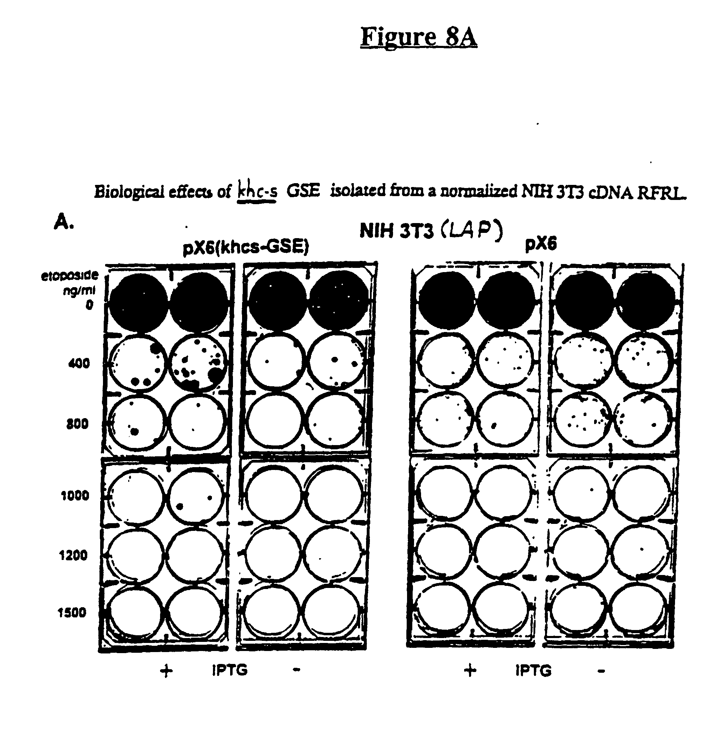

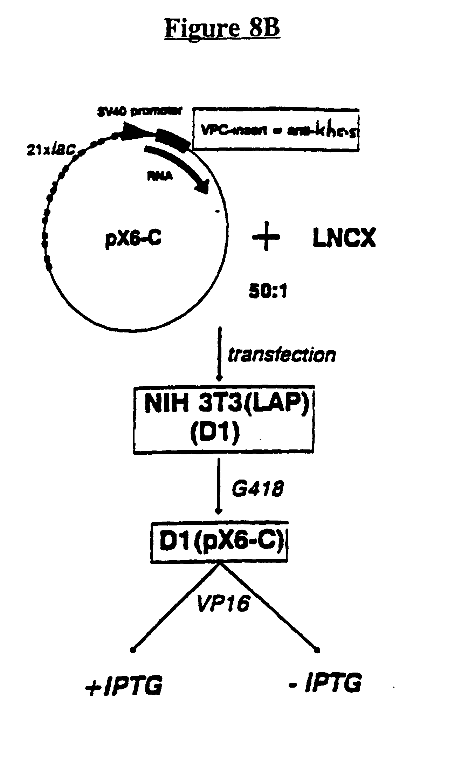

Figure 8 shows resistance to various concentrations of etoposide, conferred upon the cells by the GSE anti-*khcs* under an IPTG-inducible promoter (Panel A) and the scheme for this selection (Panel B).

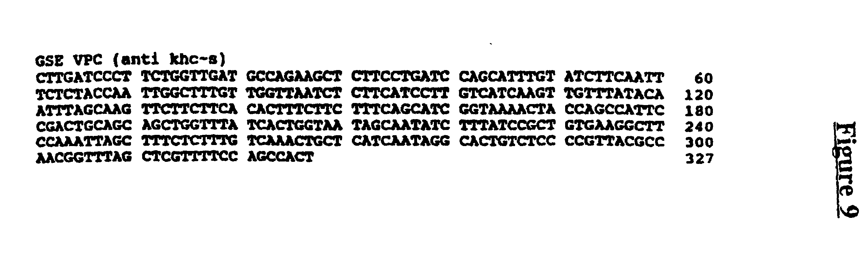

Figure 9 shows the nucleotide sequence of the GSE anti-*khcs* [SEQ ID NO 15].

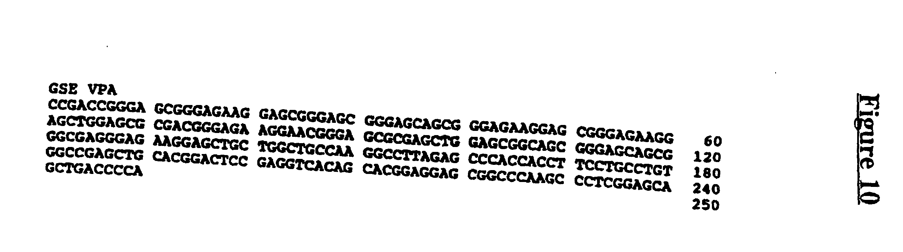

Figure 10 shows the nucleotide sequence of the GSE VPA [SEQ ID NO 16].

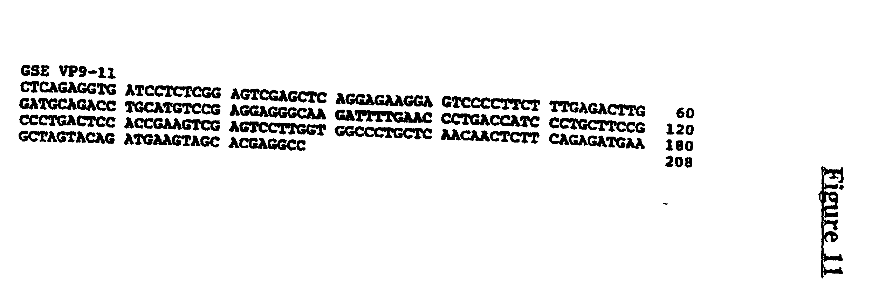

Figure 11 shows the nucleotide sequence of the GSE VP9-11 [SEQ ID NO 17].

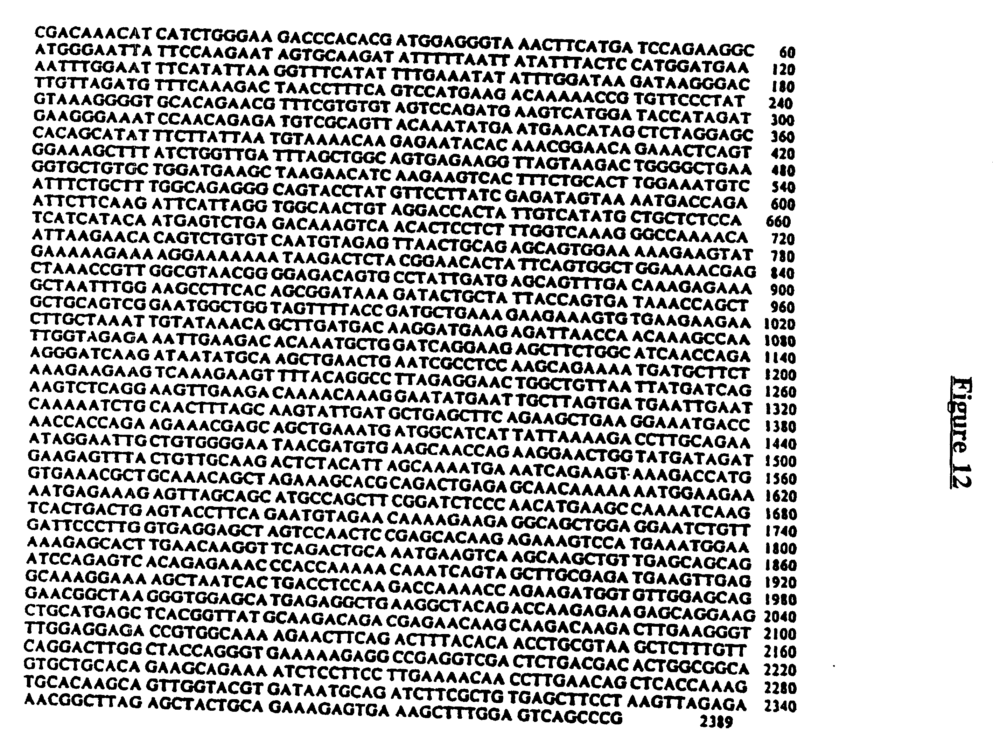

Figure 12 shows the nucleotide sequence of the most of the coding region of the mouse *khcs* cDNA [SEQ ID NO 18].

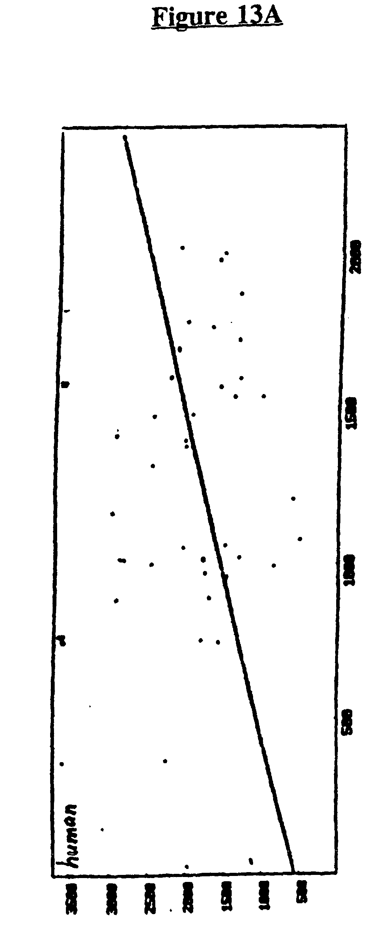

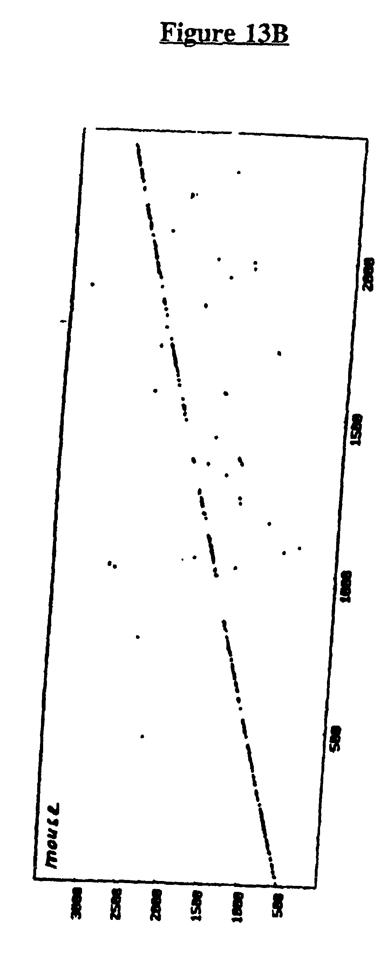

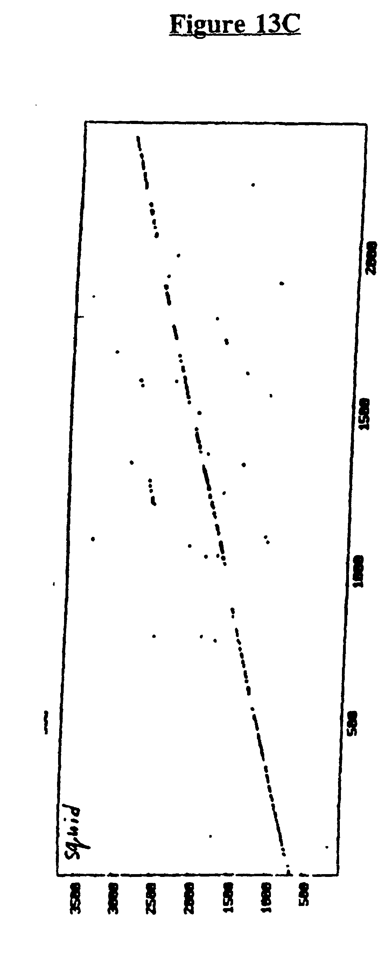

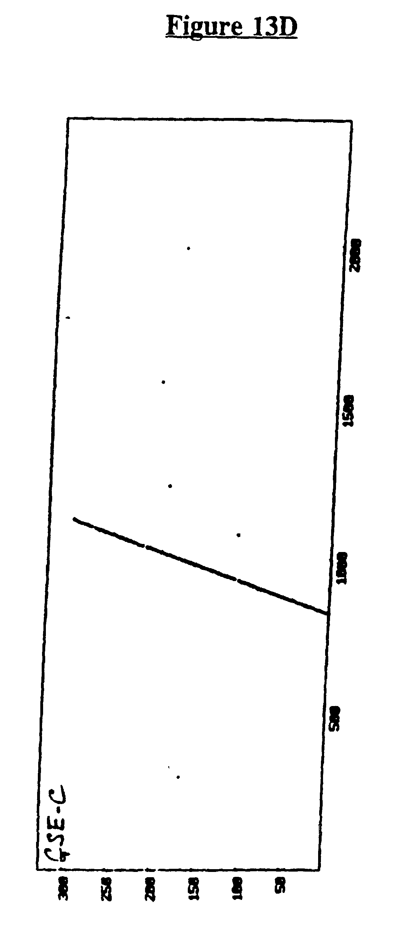

Figure 13 shows the dot matrix alignments of *khcs* protein sequence deduced from the nucleotide sequence in Figure 12 with kinesin heavy chain sequences from human (Panel A), mouse (Panel B), squid (Panel C) or the portion of mouse *khcs* encoded by the anti*-khcs* GSE (Panel D).

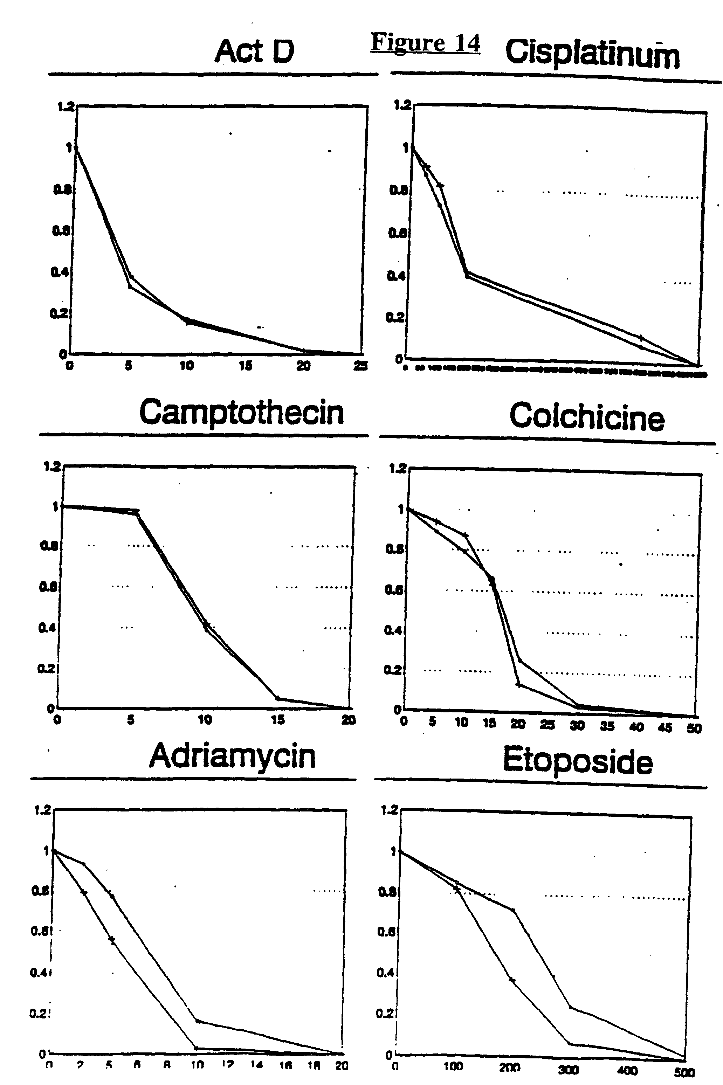

Figure 14 shows the plating efficiency in the presence of various chemotherapeutic drugs of NIH 3T3 cells infected with the LNCX vector (indicated by crosses) or with the LNCX vector containing the anti-*khcs* GSE (indicated by dots).

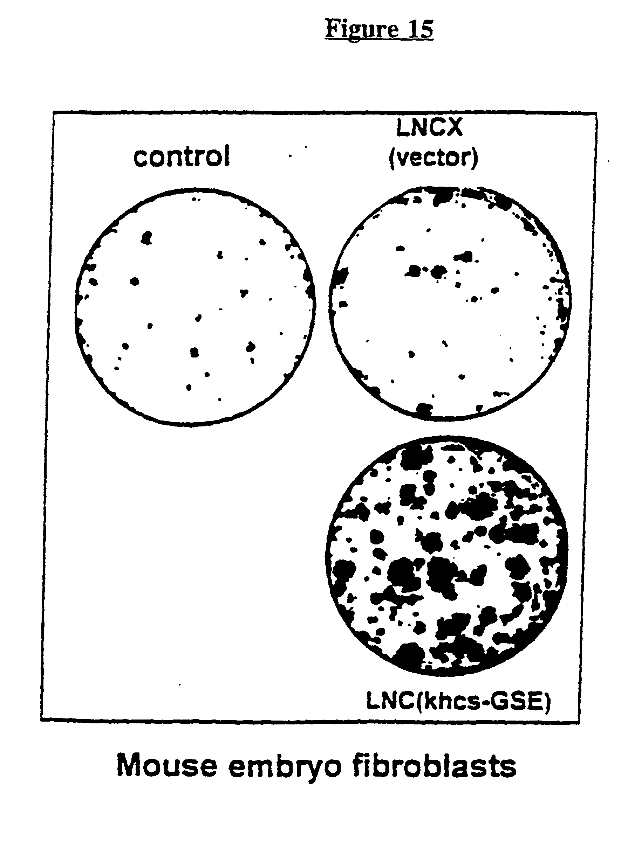

Figure 15 demonstrates increased immortalisation of primary mouse embryo fibroblasts by infection with the LNCS vector containing the anti-*khcs* GSE, relative to cells infected with the LNCS vector alone or uninfected (control) cells.

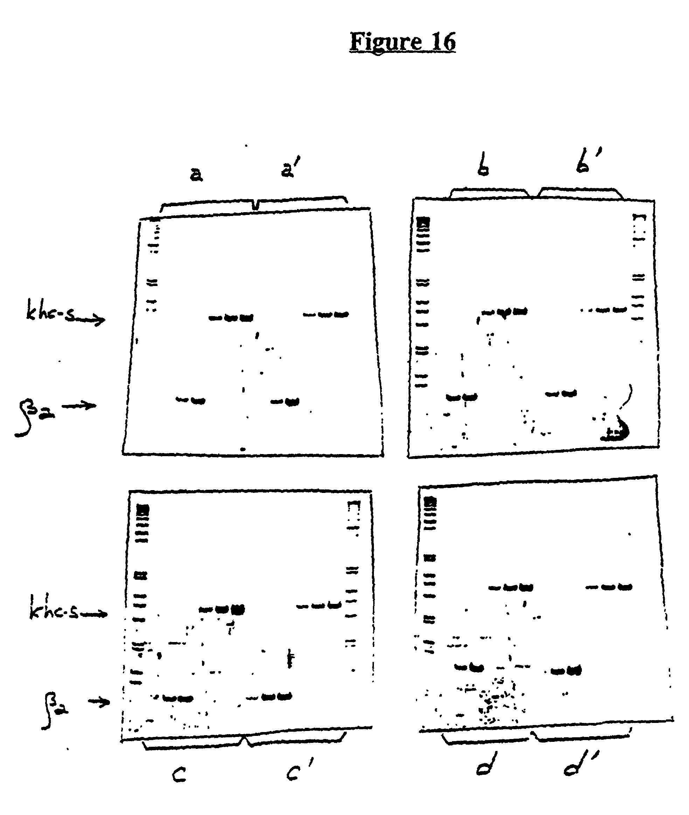

Figure 16 shows the cDNA-PCR quantitative analysis of expression of the human *khcs* gene in various unselected and etoposide-selected human HeLa cells. Lanes a shows results for clone CS(O), lanes a' for clone CX(200), lanes b for clone Σ/11(O), lanes b' for clone Σ11(1000), lanes c for clone 6(O), lanes c' for clone 6(1000), lanes d for clone Σ20(O) and lanes d' for clone Σ20(1000). The numbers in parentheses for each clone name indicate the concentration of etoposide (ng/ml) present in the growth media. Bands indicative of *khcs* expression are shown along with bands for β₂-microglobulin expression as an internal control.

### DETAILED DESCRIPTION OF THE PREFERRED EMBODIMENTS

The invention relates to means for suppressing specific gene functions that are associated with sensitivity to chemotherapeutic drugs. The invention provides genetic suppressor elements (GSEs) as defined in claim 1 that have such suppressive effect and thus confer resistance to chemotherapeutic drugs. The invention further provides methods for the use of GSEs of the invention.

GSEs of the invention can be identified according to the following method for identifying GSEs that confer resistance to any chemotherapeutic drug for which resistance is possible. The GSEs identified by this method (including the GSEs of the invention as defined by claim 1) will be homologous to a gene that is associated with sensitivity to one or more chemotherapeutic drug. For purposes of the invention, the term "homologous to a gene" has two different meanings, depending on whether the GSE acts through an antisense or antigene mechanism, or rather through mechanism of interference at the protein level. In the former case, a GSE that is an antisense or antigene oligonucleotide or polynucleotide is homologous to a gene if it has a nucleotide sequence that hybridises under physiological conditions to the gene or its mRNA transcript by Hoogsteen or Watson-Crick base-pairing. In the latter case, a GSE that interferes with a protein molecule is homologous to the gene encoding that protein molecule if it has an amino acid sequence that is the same as that encoded by a portion of the gene encoding the protein, or that would be the same, but for conservative amino acid substitutions. In either case, as a practical matter, whether the GSE is homologous to a gene is determined by assessing whether the GSE is capable of inhibiting or reducing the function of the gene.

The method comprises the step of screening a total cDNA or genomic DNA random fragment expression library phenotypically to identify clones that confer resistance to a chemotherapeutic drug. Preferably, the library of random fragments of total cDNA or genomic DNA is cloned into a retroviral expression vector. Retrovirus particles containing the library are used to infect cells and the infected cells are tested for their ability to survive in a concentration of a chemotherapeutic drug that kills uninfected cells. Preferably, the inserts in the library will range from about 100 bp to about 700 bp and more preferably, from about 200 bp to about 500 bp. Most preferably, the random fragment library will be a normalized library containing roughly equal numbers of clones corresponding to each gene expressed in the cell type from which it was made, without regard for the level of expression of any gene. However, normalization of the library is unnecessary for the isolation of GSEs that are homologous to abundantly or moderately expressed genes. Once a clonal population of cells that are resistant to the chemotherapeutic drug has been isolated, the library clone encoding the GSE is rescued from the cells. At this stage, the insert of the expression library may be tested for its nucleotide sequence. Alternatively, the rescued library clone may be further tested for its ability to confer resistance to chemotherapeutic drugs in additional transfection or infection and selection assays, prior to nucleotide sequence determination. Determination of the nucleotide sequence, of course, results in the identification of the GSE. This method is further illustrated in Examples 2-5.

Genes that are associated with sensitivity to chemotherapeutic drugs, including genes that have not been previously discovered can be identified and cloned in the following method. GSEs, or portions thereof, can be used as probes to screen full length cDNA or genomic libraries to identify their gene of origin. In some cases, genes that are associated with sensitivity to chemotherapeutic drugs will turn out to be quite surprising. For example, GSEs that abrogate etoposide sensitivity are of a particularly surprising nature. The target for etoposide is topoisomerase II, a DNA unwinding enzyme. GSEs prepared from random fragments of topoisomerase II DNA do confer resistance to etoposide. Accordingly, it would be expected that most GSEs conferring etoposide resistance would be derived from DNA encoding topoisomerase II. Surprisingly, this is not the case at all. Of three etoposide resistance-conferring GSEs obtained, two were derived from previously unidentified DNA sequences. A third such GSE was derived from the kinesin heavy chain gene. Prior to this discovery, there was no suspicion that kinesin was in any way implicated in etoposide sensitivity. These results suggest that the method will provide much new and surprising information about the genetic basis for resistance to chemotherapeutic drugs. In addition, a kinesin-derived GSE conferring resistance to etoposide caused cellular effects suggesting that kinesin may be involved in programmed cell death. If this is indeed the case, then the method also provides valuable information about the genetic basis for senescence and cell death. This may have important implications for studying genes involved in development, since GSEs used to identify genes associated with chemotherapeutic drug resistance or senescence can also be expressed as transgenes in embryos to determine the role of such genes in development. The method and its use for studying genes identified thereby and their cellular effects are further illustrated in examples 6-8.

GSEs having optimised suppressor activity for a gene associated with sensitivity to a chemotherapeutic drug may be obtained by a method in which an initial GSE is obtained by the first method described above. Then, the gene from which the GSE is derived is identified and cloned by the second method described above. This gene is then randomly fragmented and cloned into an expression vector, preferably a retroviral vector, to obtain a random fragment expression library derived exclusively from the gene of interest. This library is then transferred to and expressed in mammalian cells, which are selected in the presence of the appropriate chemotherapeutic drug. As a practical matter, such a library will contain a much greater variety of GSEs derived from the gene of interest than will a random fragment library prepared from total cDNA. Consequently, the likelihood of obtaining optimised GSEs, as determined by maximized chemotherapeutic drug resistance, from the single gene random fragment library approach is shown in greater detail in Example1.

The invention further includes synthetic peptides and oligonucleotides, as defined in claims 2 and 3, that are capable of inhibiting the function of genes associated with sensitivity to chemotherapeutic drugs. Synthetic peptides according to the invention have amino acid sequences that correspond to amino acid sequences encoded by GSEs according to the invention. Synthetic oligonucleotides according to the invention have nucleotide sequences corresponding to the nucleotide sequences of GSEs according to the invention. Once a GSE is discovered and sequenced, and its orientation is determined, it is straightforward to prepare an oligonucleotide corresponding to the nucleotide sequence of the GSE (for antisense-oriented GSEs) or amino acid sequence encoded by the GSE (for sense-oriented GSEs).

As used herein, the term oligonucleotide Includes modified oligonucleotides having nuclease-resistance internucleotide linkages such as phosphorothioate, methylphosphonate, phosphorodithioate, phosphoramidate, phosphotriester, sulfone, siloxane, carbonate, carboxymethylester, acetamidate, carbamate, thioether, bridged phosphoramidate, bridged methylene phosphonate and bridged phosphorothioate internucleotide linkages. The synthesis of oligonucleotides containing these modified linkages is well known in the art. (See e.g. Uhlmann and Peyman, 1990, Chem Rev 90: 543-584 (1990); Schneider and Banner, 1990, Tetrahedron Lett 31: 335). The term oligonucleotides also includes oligonucleotides having modified bases or modified ribose or deoxyribose sugars.

The invention also provides a diagnostic assay for tumor cells that are resistant to one or more chemotherapeutic drug due to absence of expression or under expression of a particular gene. By using the methods described herein such a gene will be identified and cloned. To determine whether absence of expression or under expression of such a gene is a naturally occurring; and thus medically significant basis for chemotherapeutic drug resistance, human tumor cells can be treated with cytotoxic quantities of an appropriate chemotherapeutic drug to select for spontaneous drug resistance mutants. These mutants can then be assessed for their level of expression of the particular gene of interest. Absence of expression or significantly reduced expression indicates a natural mechanism of chemotherapeutic drug resistance. Accordingly, such reduced or absent expression can be the basis for a diagnostic assay for tumor cell resistance to the chemotherapeutic drug or drugs of interest. A first embodiment of a diagnostic assay according to this aspect of the invention utilizes an oligonucleotide or oligonucleotides that is/are homologous to the sequence of the gene for which expression is to be measured. In this embodiment, RNA is extracted from a tumor sample, and RNA specific for the gene of interest is quantitated by standard filter hybridisation procedures, an RNase protection assay, or by quantitative cDNA-PCR. (See Noonan et al; 1990, Proc Natl Acad Sci USA 87:7160-7164). In a second embodiment of a diagnostic assay according to this aspect of the invention, antibodies are raised against a synthetic peptide having an amino acid sequence that is identical to a portion of the protein that is encoded by the gene of interest. These antibodies are then used in a conventional quantitative immunoassay (e.g. RIA or immunohistochemical assays) to determine the amount of the gene product of interest present in a sample of proteins extracted from the tumor cells to be tested, or on the surface or at locations within the tumor cells to be tested.

GSEs of the invention can be used as dominant selectable markers that are useful in gene co-transfer studies. Since GSEs according to the ' invention confer resistance to chemotherapeutic drugs, the presence of a vector that expresses the GSE can readily be selected by growth of a vector-transformed cell in a concentration of the appropriate cytotoxic drug that would be cytotoxic in the absence of the GSE. GSEs according to the invention are particularly well suited as dominant selectable markers because their small size allows them to be easily incorporated along with a gene to be cotransferred even into viral vectors having limited packaging capacity.

GSEs of the invention also provide *in vivo*-selectable markers that are useful both in gene therapy and in enhancing the effectiveness of chemotherapy. For gene therapy, GSEs according to the invention can be cotransferred on a vector into human CD34⁺ blood progenitor cells from a patient along with a therapeutic gene that, when expressed, will alleviate a genetic disorder. The cells can be selected *in vitro* for resistance to an appropriate chemotherapeutic drug, thereby assuring successful transfer of the GSE, and by implication, of the therapeutic gene as well. The progenitor cells containing the GSE and therapeutic gene can then be returned to the patient's circulation. Finally, the cells containing the GSE and therapeutic gene can be selected *in vivo* by administration of the appropriate chemotherapeutic drug (to which the GSE confers resistance) in a concentration that is cytotoxic to normal blood cells. In this way, those cells having the GSE and therapeutic gene will repopulate the patient's blood.

For enhancement of chemotherapy, a GSE according to the invention can be transferred alone or with another gene on an expression vector into CD34⁺ blood progenitor cells taken from a cancer patient. *In vitro* selection of the progenitor cells harbouring the GSE is then carried out, using the appropriate chemotherapeutic drug. The selected cells are then returned to the patient's circulation and allowed time to begin repopulating the blood. After an appropriate period, aggressive chemotherapy can be carried out, using much higher than ordinary concentrations of an appropriate chemotherapeutic drug (to which the GSE confers resistance), since toxic side effects to the immune system will be avoided due to GSE expression in those cells.

In either of these therapeutic contexts, it may be desirable to have the GSE expressed in the progenitor cells (and subsequently in the blood cells), only when its express is beneficial, i.e. during *in vivo* selection or chemotherapy. To accomplish this, an inducible promoter can be used to express the GSE. Then, the appropriate inducing agent is added to the cells prior to and during *in vitro* selection and again prior to and during *in vivo* selection or chemotherapy. As long as the inducing agent is not normally present in the human body, the GSE will not be expressed at any other time.

GSEs of the invention provide a starting point for the rational design of pharmaceutical products that can counteract resistance by tumor cells to chemotherapeutic drugs. The protein sequence encoded by genes from which the GSEs were derived can be deduced from the cDNA sequence, and the function of the corresponding proteins may be determined by searching for homology with known genes or by searching for known functional motifs in the protein sequence. If these assays do not indicate the protein function, it can be deduced through the phenotypic effects of the GSEs suppressing the gene. Such effects can be investigated at the cellular level, by analysing various growth-related, morphological, biochemical or antigenic changes associated with GSE expression. The GSE effects at the organism level can also be studied by introducing the corresponding GSEs as transgenes in transgenic animals (e.g. mice) and analysing developmental abnormalities associated with GSE expression. The gene function can also be studied by expressing the full-length cDNA of the corresponding gene, rather than a GSE, from a strong promoter in cells or transgenic animals, and studying the changes associated with overexpression of the gene.

Full length or partial cDNA sequences can also be used to direct protein synthesis in a convenient prokaryotic or eukaryotic expression system, and the produced proteins can be used as immunogens to obtain polyclonal or monoclonal antibodies. These antibodies can be used to investigate the protein localization and as specific inhibitors of the protein function, as well as for diagnostic purposes. In particular, antibodies raised against a synthetic peptide encoded by part of the complement of the sequence of the GSE anti-*khcs,* or the corresponding region of the human KHCS protein should be particularly useful, as should antibodies raised against an amino acid sequence encoded by part of the VPA or VP9-11 GSEs (see Figures 9-11).

Understanding the biochemical function of a gene involved in drug sensitivity is likely to suggest pharmaceutical means to stimulate or mimic the function of such a gene and thus augment the cytotoxic response to anticancer drugs. For example, if the gene encodes an enzyme producing a certain compound, such a compound can be synthesized chemically and administered in combination with cytotoxic drugs. If a pharmaceutical approach is not apparent from the protein function, one may be able to upmodulate gene expression at the level of transcription. This can be done by cloning the promoter region of the corresponding gene and analyzing the promoter sequence for the presence of *cis* elements known to provide the response to specific biological stimulators.

The most straightforward way to increase the expression of a drug sensitivity gene, identified through the GSE approach, would be to insert a full-length cDNA for such a gene into a retroviral vector. Such a vector, in the form of a recombinant retrovirus, will be delivered to tumor cells *in vivo,* and, upon integration, would sensitize such cells to the effects of the corresponding chemotherapeutic drug. A similar strategy for selective delivery of a drug-sensitivity gene into rat brain tumors, followed by curative treatment with the appropriate drug, was reported by Culver *et al.* (1992, *Science* 256: 1550-1552). The selective delivery to tumor cells can be accomplished on the basis of the selectivity of retrovirus-mediated transduction for dividing cells. Alternatively, the selectivity can be achieved by driving the expression of the drug sensitivity gene from a tissue-or tumor-specific promoter, such as, for example, the promoter of the carcinoembryonic antigen gene.

The protein structure decided from the cDNA sequence can also be used for computer-assisted drug design, to develop new drugs that affect this protein in the same manner as the known anticancer drugs. The purified protein, produced in a convenient expression system, can also be used as the critical component of *in vitro* biochemical screen systems for new compounds with anticancer activity. Accordingly, mammalian cells that express chemotherapeutic drug resistance-conferring GSEs according to the invention are useful for screening compounds for the ability to overcome drug resistance.

The following examples are intended to further illustrate certain preferred embodiments of the invention and are not limiting in nature.

### Example 1

### Development of GSEs For Human Topoisomerase II

Topoisomerase II is a DNA unwinding enzyme that serves as a target for many anti-cancer drugs, including etoposide, doxorubicin and amsacrine. The enzyme normally acts by double-strand DNA cleavage, followed by strand passage and religation of the breaks. Anti-cancer drugs cause trapping of the enzyme in complexes having double-strand breaks held together by the enzyme, thereby leading to lethal damage in replicating cells. Some cell lines that are resistant to anti-cancer drugs that interact with topoisomerase II have decreased expression of this enzyme.

Random fragment selection of GSEs requires transfer of the expression library into a very large number of recipient cells. Therefore, to prepare a random fragment library containing GSEs to topoisomerase II, the efficient retroviral vector system was chosen. Overlapping cDNA clones spanning the entire coding sequence for topoisomerase II were mixed and randomly fragmented to 250-350 bp fragments by DNase I in the presence of Mn⁺⁺ ions and fragment termini were filled in with T4 DNA polymerase and Klenow fragment of DNA polymerase I. After ligation with a synthetic adaptor providing translation initiation and termination codons, the fragment mixture was amplified by PCR, using adaptor-derived primers. The amplified mixture was cloned with the LNCX retroviral vector which contains a *neo* gene. (*see* Miller and Rosman, 1989, *Biotechniques* 7: 980-986).

A random fragment library containing 20,000 independent clones was obtained, and was used to transfect amphotropic and ecotropic virus-packaging cell lines derived from NIH 3T3 cells, to effect ping-pong replication-mediated amplification of the virus (*see, e.g*., Bodine *et al*., 1990, *Proc. Natl. Acad. Sci.* *USA* 87: 3738-3742). This resulted in a random fragment expression library (RFEL), a set of recombinant retroviruses containing a representative mixture of inserts derived from topoisomerase II gene sequences.

The uniformity of sequence representation in RFEL was monitored as follows. NIH 3T3 cells were infected with virus-containing supernatant, followed 24 hours later by PCR amplification of integrated proviral insert sequences in the presence of [³²P] alpha-dNTP. An aliquot of the PCR-amplified mixture was subjected to gel electrophoresis to establish the absence of predominant bands. Another aliquot was used as a probe for a Southern blot of topoisomerase II cDNA digested with several frequently cutting restriction enzymes. A representative sequence mixture was obtained, as evidenced by the absence of a predominant band in the first test, and uniform hybridization to all fragments in the second test.

RFEL was then used to infect HeLa cells, and the infectants were selected with G418. Colonies of G418-resistant cells, having about 50-70 cells each, were then exposed to etoposide at a concentration of 200 µg/ml. Approximately 50 of 10,000 G418-resistant colonies were etoposide resistant, compared to a frequency of < 10⁻⁴ when insertless retroviruses were used as a control. Cell lines were isolated from etoposide-resistant colonies. Amphotropic and ecotropic packaging cell lines producing RFEL were also selected for etoposide resistance. Virus from etoposide resistant packaging cell lines was used to infect HeLa cells, which were then selected from G418. G418-resistant infectants were challenged with three topoisomerase II-interactive anticancer drugs: etoposide, teniposide and amsacrine. A high proportion of infected cells were resistant to all three drugs, thus demonstrating that etoposide selection of mouse packaging cell lines has led to the generation of GSEs active in both human and mouse cells. These infectants were also used to establish cells lines. RFEL-derived inserts were recovered from etoposide resistant cell lines by PCR and recloned into LNCX vector. The newly-derived clones were then individually tested for the ability to confer resistance to etoposide upon transfection into HeLa cells, to confirm the GSE activity of the corresponding inserts.

Sequence analysis of 26 different isolated clones revealed that 16 of them were inserted in antisense and 10 in sense orientation. Of the 12 GSEs confirmed, 7 were sense and 5 antisense, as shown in Table I. The sequences of the confirmed GSEs are shown in Figure 1. The sense-oriented inserts of the confirmed GSEs encode 37-99 amino acid long topo II-derived peptides, initiating either from the ATG codon provided by the adaptor, or from an internal ATG codon with the open reading frame of Topoisomerase II, located close to the 5' end of the insert in an appropriate context for translation initiation. Four of the confirmed antisense GSEs come from the 3' third of the cDNA and one from the 5' end of cDNA, including the translation start site. Of the sense-oriented GSEs, five were derived from the central portion of the protein that includes the active site tyrosine-804 that covalently binds to DNA and the "leucine zipper" region involved in dimerization of Topoisomerase II. One GSE peptide is derived from the region near the N-terminus and another from the region near the C-terminus of the protein; no known functional sites are associated with either segment.

These results demonstrate the GSEs that act according to multiple mechanisms to confer etoposide resistance can be prepared from a random fragment library of DNA encoding topoisomerase II. In addition, these results show that GSEs produced from one mammalian species can be active in another mammalian species.

**Table I**

| **Confirmed Topoisomerase II-Derived GSE** | | | |
|---|---|---|---|
| Clones | Orientation (Sense/Antisense) | Position in cDNA² | Position of peptide^{b} |
| 2V | Antisense | -18 - 145 | |
| Σ11 | Sense | 393 - 605 | 134-201 |
| 6 | Sense | 2352-2532 | 808 - 844 |
| 5 | Sense | 2511-2734 | 846-911 |
| Σ28 | Sense | 2603 - 2931 | 879 - 977 |
| Σ2 | Antisense | 3150 - 3343 | |
| Σ20 | Antisense | 3486 - 3692 | |
| 39 | Antisense | 3935 - 4127 | |
| 12S | Sense | 4102 - 4343 | 1368 - 1447 |
| ΣVPs2 | Sense | 2494 - 2834 | 846 - 944 |
| Σ8 | Antisense | 4123 - 4342 | |
| ΣVM | Sense | 2501 - 2670 | 846 - 890 |

| | | | |
|---|---|---|---|
| ^{a} Position in the cDNA sequence of topoisomerase II; residues numbered as in Tsai-Pflugfelder *et al*., 1988, *Proc. Natl. Acad. Sci. USA* 85: 7177-7181. ^{b} Position of the peptide encoded by sense-oriented GSEs in the amino acid sequence of topoisomerase II; translation assumed to initiate from the first ATG codon in the correct open reading frame. | | | |

### Example 2

### Generation Of A Normalized Random Fragment cDNA Library In A Retroviral Vector

As shown in Figure 2 a normalized cDNA population was prepared using a modification of the protocol of Patanjali *et al.* (1991, *Proc. Natl. Acad. Sci. USA* 88: 1943-1947). Poly(A)⁺ RNA was extracted from NIH 3T3 cells. To obtain mRNAs for different genes expressed at various stages of the cell growth, one half of the RNA was isolated from a rapidly growing culture and the other half from quiescent cells that had reached complete monolayer confluency. To avoid overrepresentation of the 5'-end sequences in a randomly primed cDNA population, RNA was fragmented by boiling to an average size range 600-1,000 nucleotides. These RNA fragments were then used for preparing randomly primed double-stranded cDNA. This randomly primed cDNA was then ligated to a synthetic adaptor providing ATG codons in all three possible reading frames and in a proper context for translation initiation. The structure of the adaptor (see Figure 3) determined its ligation to the blunt-ended fragments of the cDNA in such a way that each fragment started from initiation codons independently from its orientation. The adaptor was not supplied with termination codons in the opposite strand since the cloning vector pLNCX, contained such codons immediately downstream of the cloning site. This vector has been described by Miller and Rosman (1989, *Biotechniques* 7: 980-986). The litigated mixture was amplified by PCR, using the "sense" strand of the adaptor as a PCR primer, in contrast to the method of Patanjali et al., which utilized cloning the initial cDNA preparation into a phage vector and then using vector-derived sequences as PCR primers to amplify the cDNA population. The PCRs were carried out in 12 separate reactions that were subsequently combined, to minimize random over- or under-amplification of specific sequences and to increase the yield of the product. The PCR-amplified mixture was size-fractionated by gel electrophoresis, and 200-500 bp fragments were selected for subsequent manipulations in contrast to Patanjali's fragment size range of from 400 to 1,600 bp.

For normalization, the cDNA preparation was denatured and reannealed, using different time points, as described by Patanjali *et al., supra,* and shown in Figure 2, for reannealing. The single-stranded and double-stranded DNAs from each reannealed mixture were separated by hydroxyapatite chromatography. The single-stranded DNA fractions from each time point of reannealing were PCR-amplified using the adaptor-derived primer and analyzed by Souther hybridization for the relative abundance of different mRNA sequences. The fraction that contained similar proportions of tubulin, *c-myc* and *c-fos* cDNA sequences (see Figure 2), corresponding to medium- and low-expressed genes, respectively, was used for the library preparation.

The normalized cDNA preparation was cloned into the *Cla*I site of the MoMLV-based retroviral vector pLNCX, which carries the *neo* (G418 resistance) gene, transcribed from the promoter contained in the retroviral long terminal repeat (LTR), and which expresses the inserted sequence from a strong promoter of the cytomegalovirus (CMV) (see Figure 3). The ligation mixture, divided into five portions, was used for five subsequent large-scale transformations of *E. coli.* The transformed bacteria was plated on the total of 500 agar plates (150 mm in diameter) and the plasmid population (18 mg total) was isolated from the colonies washed off the agar. A total of approximately 5 x 10⁷ clones were obtained, more than 60% of which carried the inserts of normalized cDNA, as estimated by PCR amplification of inserts from 50 randomly picket colonies. These results demonstrate the feasibility of generating a normalized cDNA library of as many as 3 x 10⁷ recombinant clones in a retroviral plasmid expression vector.

### Example 3

### Transduction Of A Retroviral Random Fragment Library Into Virus-Packaging Cell Lines and NIH 3T3 Cells

The plasmid library prepared according to Example 2 was converted into a mixture of retroviral particles by transfection into virus-packaging cells (derivatives of NIH 3T3) that express retroviral virion proteins. Examples of such cell lines have been described by Markowitz *et al.* (1988, *Virology* 167: 400-406). Ecotropic and amphotropic virus-packaging cell lines, GP + E86 and GP + envAm12, respectively, were mixed at 1:1 ratio, and 10⁷ cells of this mixture were transfected with the plasmid library under standard calcium phosphate coprecipitation conditions. This transfection resulted in the packaging and secretion of ecotropic and amphotropic virus particles, which rapidly spread through the packaging cell population, since ecotropic viruses are capable of infecting amphotropic packaging cells and vice versa. The yield of the virus, as measured by the number of G418-resistant colonies obtained after the infection of NIH 3T3 cells, reached 10⁵ infectious units per 1 ml of media during the stage of transient transfection (1-3 days), then decreased (4-8 days) and then rapidly increased due to the expression of proviral genomes that became stably integrated in most of the packaging cells. The yield of the virus 9-12 days after transfection reached > 10⁶ per 1 ml of media supernatant. At this stage, the library showed fairly even representation of different fragments, but at later stages individual virus-producing clones began to predominate in the population, leading to uneven representation of cDNA-derived inserts. The uniformity of sequence representation in the retroviral population was monitored by rapid extraction of DNA from cells infected with the virus-containing supernatant, followed by PCR amplification of inserts. The inserts were analyzed first by the production of a continuous smear in ethidium-bromide stained agarose gel and then by Southern hybridization with different probes, including topoisomerase II, *c-myc* and tubulin. As long as each gene was represented by a smear of multiple fragments, the representativity of the library was considered to be satisfactory.

In other experiments, for transducing the random-fragmented normalized cDNA library into NIH 3T3 cells, without loss of representativity, NIH 3T3 cells were infected either with a virus produced at the transient stage of transfection (days 1-3), or with the high-titer virus collected 10-12 days after transfection. In the latter case, 100 ml of viral suspension contained more than 10⁸ infectious units. In the case of the "transient" virus, NIH 3T3 cells were infected with at least 10⁷ recombinant retroviruses by using 500 ml of media from virus-producing cells (five rounds of infection, 100 ml of media in each). These results demonstrate the feasibility of converting a large and complex random fragment library into retroviral form and delivering it to a non-packaging cell line without loss of complexity.

### Example 4

### Isolation of GSEs Conferring Resistance To The Chemotherapeutic Drug Etoposide

The overall scheme for the selection of GSEs conferring etoposide resistance is illustrated in Figure 4. This selection was carried out directly on virus-producing packaging cells, in the expectation that cells whose resistant phenotype is caused by the GSE expression will produce virus particles carrying such a GSE. The mixture of amphotropic and ecotropic packaging cells was transfected with the cDNA library in the LNCX vector, prepared according to Example 2 and the virus was allowed to spread through the population for 9 days. Analysis of a small part of the population for G418 resistance showed that practically 100% of the cells carried the *neo*-containing provirus. The cells were then exposed to 350 ng/ml etoposide for 15 days and then allowed to grow without drug for two more weeks. No difference was observed between the numbers of colonies obtained in the experiment and in the control (uninfected cells or cells infected with the insert-free LNCX virus) after etoposide selection. The virus present in the media supernatant of the surviving cells was then used to infect NIH 3T3 cells followed by etoposide selection using essentially the same protocol. NIH 3T3 cells infected with the library-derived virus produced by packaging cells that were selected with etoposide showed a major increase in the number of etoposide-resistant cells relative to the control cells infected with the insert-free LNCX virus, indicating the presence of biologically active GSEs in the preselected virus population (see Figure 5A).

The proviral inserts contained in the etoposide-selected NIH 3T3 cells were analyzed by PCR. This analysis (see Figure 5B) showed an enrichment for specific fragments, relative to the unselected population of the infected cells. Individual PCR-amplified fragments were recloned into the LNCX vector in the same position and orientation as in the original plasmid, as illustrated in Figure 6. A total of 42 proviral inserts, enriched after etoposide selection, were thus recloned, and tested either in batches or individually for the ability to confer increased etoposide resistance after retroviral transduction into NIH 3T3 cells. Three non-identical clones were found to induce etoposide resistance, indicating that they contained biologically active GSEs. Etoposide resistance induced by these clones is illustrated in Figures 7 and 8. These GSEs were named anti-*khcs*, VPA and VP9-11.

The ability of one of these GSEs (anti-*khcs*) to induce etoposide resistance was further documented by using the isopropyl β-D-thiogalactopyranoside (IPTG)-inducible promoter/activator system, as described by Baim *et al.* (1991, *Proc. Natl. Acad. Sci. USA* 88: 5072-5076). The components of this system include an enhancer-dependent promoter, combined in cis with multiple repeats of the bacterial *lac* operator, and a gene expressing LAP265, an artificial regulatory protein derived from the *lac* repressor and a mammalian transcriptional activator. The anti-*khcs* GSE was cloned into the plasmic pX6.CLN, which contains the inducible promotor used by Baim *et al., supra,* (a gift of Dr. T. Shenk) which expresses the inserts from an enhancerless SV40 early gene promoter supplemented with 21 repeats of the *lac* operator sequence. The resulting plasmid, which contains no selectable markers, was co-transfected into NIH 3T3 cells together with the LNCX plasmid carrying the *neo* gene. The mass population of G418-selected transfectants, along with control cells transfected with the insert-free vector, was exposed to increasing concentrations of etoposide, in the presence or in the absence of 5 mM IPTG. Even though the co-transfection protocol usually leads to the integration of the GSE in only a fraction of the G418-resistant cells, transfection with anti-*khcs* resulted in a clearly increased etoposide resistance, which was dependent on IPTG (see Figure 8).

### Example 5

### Sequence Analysis of GSEs Conferring Resistance To The Chemotherapeutic Drug Etoposide

The GSEs anti-*khcs,* VPA, and VP9-11, cloned as described in Example 4, were sequenced by the standard dideoxy sequencing procedure, and the deduced sequences are shown in Figure 9-11. The nucleotide sequences of the "sense" and "antisense" strands, as well as amino acid sequences of the peptides encoded by these strands, were analyzed for homology to the nucleic acid and protein sequences present in the National Center for Biotechnology Information data base, using the BLAST network program for homology search. No significant homology with any sequence was detected for GSEs VPA and VP9-11. In contrast, the sequence corresponding to the "antisense" strand of the anti-*khcs* GSE, showed strong homology with several genes encoding the heavy chain of kinesins, a family of microtubule motor proteins involved in intracellular movement of organelles or macromolecules along the microtubules of eukaryotic cells. This protein family has been reviewed by Endow (1991, *Trends Biochem. Sci.* 16: 221-225). The highest homology was found with the human kinesin heavy chain (KHC) gene, as described by Navone *et al.* (1992, *J. Cell Biol.* 117: 1263-1275). Anti-*khcs* therefore encodes antisense RNA for a mouse *khc* gene, which we term *khcs* for *khc* associated with sensitivity (to drugs) or senescence. We refer to the kinesin molecule, formed by the associate of the Khcs protein with kinesin light chains, as kinesin-S, to distinguish it from the other kinesins present in mammalian cells. These results demonstrate that chemotherapeutic drug selection for GSEs can lead to the discovery of novel genetic elements, and can also reveal roles of genes in drug sensitivity that had never before been suspected.

### Example 6

### Cloning And Analysis Of The Gene From Which Anti-khcs GSE Gene Was Derived

The anti-*khcs* GSE isolated in Example 4 was used as a probe to screen 400,000 clones from each of two cDNA libraries in the lambda gt10 vector. These libraries were prepared by conventional procedures from the RNA of mouse BALB/c 3T3 cells, either unsynchronized or at Go -> G1 transition, as described by Lau and Nathans (1985, *EMBO J.* 4: 3145-3151 and 1987, *Proc. Natl. Acad. Sci. USA* 84: 1182-1186, a gift of Dr. Lau). Screening of the first library yielded no hybridizing clones, but two different clones from the second library were found to contain anti-*khcs* sequences. These clones were purified and sequenced. Sequence analysis showed that we have isolated the bulk of the mouse *khcs* cDNA, corresponding to 796 codons (the full-length human KHC cDNA encodes 963 amino acids). This sequence is shown in Figure 12. The missing 5' and 3' terminal sequences are currently being isolated using the "anchored PCR" technique, as described by Ohara *et al.* (1989, *Proc. Natl. Acad. Sci. USA* 86: 5763-5677).

The dot-matrix alignment of the sequenced portion of the *khcs* protein with previously cloned KHC proteins from the human (*see* Navone *et al.,* 1992, *J. Cell. Biol.* 117:1263-1275), mouse (*see* Kato, 1991, *J. Neurosci.* 2: 704-711) and squid (*see* Kosik *et al.,* 1990, *J. Biol. Chem.* 265: 3278-3283) is shown in Figure 13. The portion corresponding to the anti-*khcs* GSE, is shown in Figure 9. The *khcs* gene is most highly homologous to the human gene (97% amino acid identity), suggesting that the human KHC (KHCS) gene is functionally equivalent to the mouse *khcs*. The alignment also shows that the anti-*khcs* GSE corresponds to the region which is the most highly diverged between different kinesins. This result suggests that the anti-*khcs* GSE is an antisense-oriented GSE fragment with an inhibitory effect specific to kinesin-S and not to the other mouse kinesins. This suggests the possibility that suppression of the other members of the kinesin family would have a detrimental effect on cell growth, thus resulting in selective isolation of the most specific sequence within the *khcs* gene.

### Example 7

### Assessment Of Drug Cross-Resistance Conferred By Resistance To Etoposide

To determine the spectrum of drugs to which the anti-*khcs* GSE would confer resistance, we have developed a stable population of ecotropic packaging cells producing the LNCX virus with the *anti-khcs* insert. This virus was used to infect NIH 3T3 cells. Two days after infection, the cells were analyzed for resistance to several different drugs, relative to control cells infected with the LNCX vector virus, using the standard plating efficiency assay. Figure 14 shows one out of three sets of experiments carried out with different drugs by this assay. Cells infected with the anti-*khcs* virus showed a clear increase in their resistance to etoposide and Adriamycin relative to control NIH 3T3 cells infected with the control LNCX virus. No changes in resistance were observed with colchicine, cisplatin, camptothecin, or actinomycin D. These latter results remain preliminary, however, because this assay, analyzing total unselected virus-infected populations is relatively insensitive, compared with analysis of highly expressing individually-selected clones, Overall, these results demonstrate that selection of GSEs that confer resistance to one chemotherapeutic drug can result in obtaining GSEs that confer resistance to additional chemotherapeutic drugs.

### Example 8

### Assessment of Cellular Effects Of GSEs That Confer Resistance To Etoposide

The virus carrying the anti-*khcs* GSE was tested for the ability to increase the life span of primary mouse embryo fibroblasts (MEF). MEF were prepared from 10 day old mouse embryos by a standard trypsinization procedure and senescent cells were frozen at different passages prior to crisis. Senescent MEF, two weeks before crisis, were infected with recombinant retroviruses carrying LNCX vector either without an insert or with anti-*khcs*. Figure 15 shows MEF cell colonies two weeks after crisis. Relative to uninfected MEF cells, or cells infected with a control LNCX virus, cells infected with the anti-*khcs* showed a great increase in the proportion of cells surviving the crisis. Post-crisis cells infected with the anti*-khcs* virus showed no microscopically visible features of neoplastic transformation. These results indicate that anti-*khcs* promotes the immortalization of normal senescent fibroblasts. These results suggest that the normal function of kinesin-S may be associated with the induction of programmed cell death occurring after exposure to certain cytotoxic drugs or in the course of cellular senescence. These results also indicate that isolation of GSEs that confer resistance to chemotherapeutic drugs can provide insight into the cellular genes and processes involved in cell growth regulation.

### Example 9

### Assessment Of The Role Of Decreased khcs Gene Expression In Naturally Occurring Mechanisms Of Drug Resistance

To test whether decreased *khcs* gene expression is associated with any naturally occurring mechanisms of drug resistance, an assay was developed for measuring *khcs* mRNA levels by cDNA-PCR. This assay is a modification of the quantitative assay described by Noonan *et al.* (1990, *Proc. Natl. Acad. Sci. USA* 87: 7160-7164) for determining *mdr-1* gene expression. The oligonucleotide primers had the sequences AGTGGCTGGAAAACGAGCTA [SEQ. ID. NO. 19] and CTTGATCCCTTCTGGTTGAT [SEQ. ID. NO. 20]. These primers were used to amplify a 327 bp segment of mouse *khcs* cDNA, corresponding to the anti-*khcs* GSE. These primers efficiently amplified the mouse cDNA template but not the genomic DNA, indicating that they spanned at least one intron in the genomic DNA. Using these primers, we determined that *khcs* mRNA is expressed at a higher level in the mouse muscle tissue than in the kidney, liver or spleen.

In another experiment a pair of primers amplifying a homologous segment of the human KHCS cDNA was selected, based on the reported human KHC sequence published by Navone *et al.* (1992, *J. Cell. Biol.* 117: 1263-1275). The sequences of these primers are AGTGGCTTGAAAATGAGCTC [SEQ. ID. NO. 21] and CTTGATCCCTTCTGGTAGATG [SEQ. ID. NO. 22], and they amplify a 327 bp cDNA fragment. These primers were used to test for changes in the KHCS gene expression in several independently isolated populations of human HeLa cells, each selected for spontaneously acquired etoposide resistance. β₂-microglobulin cDNA sequences were amplified as an internal control. Figure 16 shows the results of the cDNA-PCR assay on the following populations: CX(0), HeLa population infected with the LNCX vector virus and selected with G418; CX (200), the same cells selected for resistance to 200 ng/ml etoposide; Σ11(O), 6(O) and Σ21(O), populations obtained after infection of HeLa cells with recombinant retroviruses carrying different GSEs derived from topoisomerase α cDNA, as described in Example 1, and selected with G418; Σ11(1000), 6(1000) and Σ21(1000), the same populations selected for resistance to 1 µg/ml etoposide. As shown in Figure 16, the yield of the PCR product specific for the *khcs* gene was significantly lower in each of the etoposide-selected populations than in the control cells. This result indicates that a decrease in the *khcs* gene expression is a common natural mechanism for drug resistance.

## Claims

1. A GSE having a nucleotide sequence as shown in Figure 9 (SEQ ID NO.15), Figure 10 (SEQ ID NO.16) or Figure 11 (SEQ ID NO.17) or its complement.

2. A synthetic peptide having an amino acid sequence corresponding to the amino acid sequence encoded by a GSE as claimed in claim 1.

3. A synthetic oligonucleotide having a nucleotide sequence of an antisense RNA encoded by a GSE as claimed in claim 1.

4. The use of a GSE as claimed in claim 1 for conferring etoposide resistance on a eukaryotic cell in vitro.

5. The use of a GSE as claimed in claim 1 in a method of isolating a gene associated with sensitivity to a chemotherapeutic drug comprising the step of screening a cDNA library with the GSE.

6. The use of a GSE as claimed in claim 1 for diagnosing chemotherapeutic drug resistance in a tumour sample, the use comprising the step of assaying the tumour sample for expression of a gene that hybridises to the GSE wherein drug resistant tumour samples have reduced or absent expression of said gene.

7. A mammalian cell that expresses a GSE as claimed in claim 1.

8. An antibody reactive to a protein or polypeptide encoded by a GSE as claimed in claim 1.

9. The use of a GSE as claimed in claim 1 for the manufacture of a medicament for the treatment of cancer.

10. A GSE as claimed in claim 1 for use as a pharmaceutical.

11. The use of a GSE as claimed in claim 1 to identify genes associated with chemotherapeutic drug resistance or senescence.

12. The use of a GSE as claimed in claim 1 for the manufacture of a medicament for enhancing chemotherapy.

13. The use as claimed in claim 12, wherein the medicament comprises chemotherapeutic drug resistant blood progenitor cells.

## Patentansprüche

1. GSE mit einer Nukleotidsequenz, wie sie in Figur 9 (SEQ ID NO.15), Figur 10 (SEQ ID NO.16) oder Figur 11 (SEQ ID NO.17) gezeigt ist, oder die komplementär dazu ist.

2. Synthetisches Peptid mit einer Aminosäuresequenz, die der Aminosäuresequenz entspricht, für die ein GSE gemäß Anspruch 1 kodiert.

3. Synthetisches Oligonukleotid, mit einer Nukleotidsequenz einer Antisense-RNA, für die ein GSE gemäß Anspruch 1 kodiert.

4. Verwendung eines GSE gemäß Anspruch 1, um einer eukaryontischen Zelle in vitro eine Etoposid-Resistenz zu verleihen.

5. Verwendung eines GSE gemäß Anspruch 1 in einem Verfahren zur Isolierung eines Gens, das mit einer Sensibilität gegenüber einem chemotherapeutischen Wirkstoff im Zusammenhang steht, umfassend den Schritt des Screenings einer cDNA-Bibliothek mit dem GSE.

6. Verwendung eines GSE gemäß Anspruch 1 zur, Diagnose von Resistenz gegen einen chemotherapeutischen Wirkstoff in einer Tumorprobe, wobei die Verwendung den Schritt des Untersuchens der Tumorprobe auf die Expression eines Gens, das mit dem GSE hybridisiert, umfasst, wobei Wirkstoff-resistente Tumorproben eine verringerte oder keine Expression des Gens aufweisen.

7. Säugerzelle, die ein GSE gemäß Anspruch 1 exprimiert.

8. Antikörper, der gegenüber einem Protein oder Polypeptid, für das ein GSE gemäß Anspruch 1 kodiert, reaktiv ist.

9. Verwendung eines GSE gemäß Anspruch 1 zur Herstellung eines Medikaments für die Behandlung von Krebs.

10. GSE nach Anspruch 1 zur Verwendung als ein Pharmazeutikum.

11. Verwendung eines GSE gemäß Anspruch 1, um Gene zu identifizieren, die mit einer Resistenz gegen einen chemotherapeutischen Wirkstoff oder mit dem Altern im Zusammenhang stehen.

12. Verwendung eines GSE gemäß Anspruch 1 zur Herstellung eines Medikaments zur Verbesserung von Chemotherapie.

13. Verwendung nach Anspruch 12, wobei das Medikament Blutvorläuferzellen umfasst, die gegen einen chemotherapeutischen Wirkstoff resistent sind.

## Revendications

1. GSE ayant une séquence nucléotidique telle que représentée à la figure 9 (SEQ ID N° 15), figure 10 (SEQ ID N° 16) ou figure 11 (SEQ ID N° 17) ou son complémentaire.

2. Peptide synthétique ayant une séquence d'acides aminés correspondant à la séquence d'acides aminés codée par un GSE tel que revendiqué à la revendication 1.

3. Oligonucléotide synthétique ayant une séquence nucléotidique d'un ARN antisens codé par un GSE tel que revendiqué à la revendication 1.

4. Utilisation d'un GSE tel que revendiqué à la revendication 1 pour conférer une résistance à l'étoposide dans une cellule eucaryote *in vitro.*

5. Utilisation d'un GSE tel que reven diqué à la revendication 1 dans une méthode d'isolement d'un gène associé à une sensibilité à une drogue chimiothérapeutique comprenant l'étape de criblage d'une banque d'ADNc avec le GSE.

6. Utilisation d'un GSE tel que revendiqué à la revendication 1 pour le diagnostic d'une résistance à une drogue chimiothérapeutique dans un échantillon tumoral, l'utilisation comprenant l'étape consistant à tester dans l'échantillon tumoral l'expression d'un gène qui hybride au GSE, les échantillons tumoraux résistants à la drogue ayant une expression réduite ou n'ayant aucune expression dudit gène.

7. Cellule de mammifère qui exprime un GSE tel que revendiqué à la revendication 1.

8. Anticorps réactif envers une protéine ou un polypeptide codé par un GSE tel que revendiqué à la revendication 1.

9. Utilisation d'un GSE tel que revendiqué à la revendication 1 pour la fabrication d'un médicament pour le traitement du cancer.

10. GSE tel que revendiqué à la revendication 1 pour l'utilisation comme produit pharmaceutique.

11. Utilisation d'un GSE tel que revendiqué à la revendication 1 pour identifier des gènes associés à une résistance à une drogue chimiothérapeutique ou à une sénescence.

12. Utilisation d'un GSE tel que revendiqué à la revendication 1 pour la fabrication d'un médicament pour améliorer une chimiothérapie.

13. Utilisation selon la revendication 12, dans laquelle le médicament comprend des cellules progénitrices sanguines résistantes à une drogue chimiothérapeutique.


### SEQUENCE LISTING

(1) GENERAL INFORMATION:
   (i) APPLICANT:
      (A) NAME: Board of Trustees of the University of Illinois
      (B) STREET: 352 Henry Administration Building, 506 South Wright Street
      (C) CITY: Urbana
      (D) STATE: Illinois
      (E) COUNTRY: USA
      (F) POSTAL CODE (ZIP): 61801
      (G) TELEPHONE:
      (H) TELEFAX:
   (ii) TITLE OF INVENTION: Genes And Genetic Elements Associated
      With Sensitivity To Chemotherapeutic Drugs
   (iii) NUMBER OF SEQUENCES: 22
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25 (EPO)
   (v) CURRENT APPLICATION DATA:
      APPLICATION NUMBER: PCT/US94/
(2) INFORMATION FOR SEQ ID NO:1:
   (i) SEQUENCE CHARACTERISTICS:
      (A) LENGTH: 164 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: YES
   (xi) SEQUENCE DESCRIPTION: SEQ ID NO:1:
   GTGTCTGGGC GGAGCAAAAT ATGTTCCAAT TGTGTTTTCT TTTGATAGAT TCTTTCAACA 60
   GACAGTCTTT TCTTAGCATC TTCATTTTTC TTTATTTTGT TGACTTGCAT ATTTTCATTT 120
   ACAGGCTGCA ATGGTGACAC TTCCATGGTG ACGGTCGTGA AGGG 164
(2) INFORMATION FOR SEQ ID NO:2:
   (i) SEQUENCE CHARACTERISTICS:
      (A) LENGTH: 213 base pairs
      (B) TYPE: nucleic acid
      (C) STRANDEDNESS; single
      (D) TOPOLOGY: linear
   (ii) MOLECULE TYPE: cDNA
   (iii) HYPOTHETICAL: NO
   (iv) ANTI-SENSE: YES
   (xi) SEQUENCE DESCRIPTION: SEQ ID NO:2:
   TGAAAAGATG TATGTCCCAG CTCTCATATT TGGACAGCTC CTAACTTCTA GTAACTATGA 60
   TGATGATGAA AAGAAAGTGA CAGGTGGTCG AAATGGCTAT GGAGCCAAAT TGTGTAACAT 120
   ATTCAGTACC AAATTTACTG TGGAAACAGC CAGTAGAGAA TACAAGAAAA TGTTCAAACA 180
   GACATGGATG GATAATATGG GAAGAGCTGG TGA 213
(2) INFORMATION FOR SEQ ID NO:3:
   (i) SEQUENCE CHARACTERISTICS:
      (A) LENGTH: 181 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: YES
   (xi) SEQUENCE DESCRIPTION: SEQ ID NO:3:
   GCCCATTGGT CAGTTTGGTA CCAGGCTACA TGGTGGCAAG GATTCTGCTA GTCCACGATA      60
   CATCTTTACA ATGCTCAGCT CTTTGGCTCG ATTGTTATTT CCACCAAAAC ATGATCACAC     120
   GTTGAAGTTT TTATATGATG ACAACCAGCG TGTTGAGCCT GAATGGTACA TTCCTATTAT     180
   T                                                                     181
(2) INFORMATION FOR SEQ ID NO:4:
   (i) SEQUENCE CHARACTERISTICS:
      (A) LENGTH: 224 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: YES
   (xi) SEQUENCE DESCRIPTION: SEQ ID NO:4:
   TGAATGGTAC ATTCCTATTA TTCCCATGGT GCTGATAAAT GGTGCTGAAG GAATCGGTAC      60
   TGGGTGGTCC TGCAAAATCC CCAACTTTGA TGTGCGTGAA ATTGTAAATA ACATCAGGCG     120
   TTTGATGGAT GGAGAAGAAC CTTTGCCAAT GCTTCCAAGT TACAAGAACT TCAAGGGTAC     180
   TATTGAAGAA CTGGCTCCAA ATCAATATGT GATTAGTGGT GAAG                      224
(2) INFORMATION FOR SEQ ID NO:5:
   (i) SEQUENCE CHARACTERISTICS:
      (A) LENGTH: 329 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: YES
   (xi) SEQUENCE DESCRIPTION: SEQ ID NO:5:
   TGCGTGAAAT TGTAAATAAC ATCAGGCGTT TGATGGATGG AGAAGAACCT TTGCCAATGC 60
   TTCCAAGTTA CAAGAACTTC AAGGGTACTA TTGAAGAACT GGCTCCAAAT CAATATGTGA 120
   TTAGTGGTGA AGTAGCTATT CTTAATTCTA CAACCATTGA AATCTCAGAG CTTCCCGTCA 180
   GAACATGGAC CCAGACATAC AAAGAACAAG TTCTAGAACC CATGTTGAAT GGCACCGAGA 240
   AGACACCTCC TCTCATAACA GACTATAGGG AATACCATAC AGATACCACT GTGAAATTTG 300
   TTGTGAAGAT GACTGAAGAA AAACTGGCA 329
(2) INFORMATION FOR SEQ ID NO:6:
   (i) SEQUENCE CHARACTERISTICS:
      (A) LENGTH: 194 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: YES
   (xi) SEQUENCE DESCRIPTION: SEQ ID NO:6:
   CACTCTTTTC AGTTTCCTTT TCGTTGTCAC TCTCTTCATT TTCTTCTTCA TCTGGAACCT 60
   TTTGCTGGGC TTCTTTCCAG GCCTTCACAG GATCCGAATC ATATCCCCTC TGAATCAGAA 120
   CTTTAATTAA TTCTTTCTTA GGCTTATTTT CAATGATTAT TTTGCCATCT ATTTTCTCAT 180
   AGATAAAGCG AGCC 194
(2) INFORMATION FOR SEQ ID NO:7:
   (i) SEQUENCE CHARACTERISTICS:
      (A) LENGTH: 206 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: YES
   (xi) SEQUENCE DESCRIPTION: SEQ ID NO:7:
   TCTGCCTCTG CTTTCATTTC TATGGTTATT CGTGGAATGA CTCTTTGACC ACGCGGAGAA 60
   GGCAAAACTT CAGCCATTTG TGTTTTTTTC CCCTTGGCCT TCCCCCCTTT CCCAGGAAGT 120
   CCGACTTGTT CATCTTGTTT TTCCTTGGCT TCAACAGCCT CCAATTCTTC AATAAATGTA 180
   GCCAAGTCTT CTTTCCACAA ATCTGA 206
(2) INFORMATION FOR SEQ ID NO:8:
   (i) SEQUENCE CHARACTERISTICS:
      (A) LENGTH: 194 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: YES
   (xi) SEQUENCE DESCRIPTION: SEQ ID NO:8:
   GACACGACAC TTTTCTGTGG TTTCAGTTCT TTGTTACTAA GTTTTGGGGA AGTTTTGGTC 60
   TTAGGTGGAC TAGCATCTGA TGGGACAAAA TCTTCATCAT CAGTTTTTTC ATCAAAATCT 120
   GAGAAATCTT CATCTGAATC CAAATCCATT GTGAATTTTG TTTTTGTTGC TGCTCTCCGT 180
   GGCTCTGTTT CTCG 194
(2) INFORMATION FOR SEQ ID NO:9:
   (i) SEQUENCE CHARACTERISTICS:
      (A) LENGTH: 242 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: YES
   (xi) SEQUENCE DESCRIPTION: SEQ ID NO:9:
   CTGAAACCAC AGAAAAGTGT CGTGTCAGAC CTTGAAGCTG ATGATGTTAA GGGCAGTGTA 60
   CCACTGTCTT CAAGCCCTCC TGCTACACAT TTCCCAGATG AAACTGAAAT TACAAACCCA 120
   GTTCCTAAAA AGAATGTGAC AGTGAAGAAG ACAGCAGCAA AAAGTCAGTC TTCCACCTCC 180
   ACTACCGGTG CCAAAAAAAG GGCTGCCCCA AAAGGAACTA AAAGGGATCC AGCTTTGAAT 240
   TC 242
(2) INFORMATION FOR SEQ ID NO:10:
   (i) SEQUENCE CHARACTERISTICS:
      (A) LENGTH: 341 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: YES
   (xi) SEQUENCE DESCRIPTION: SEQ ID NO:10:
   AACCAGCGTG TTGAGCCTGA ATGGTACATT CCTATTATTC CCATGGTGCT GATAAATGGT 60
   GCTGAAGGAA TCGGTACTGG GTGGTCCTGC AAAATCCCCA ACTTTGATGT GCGTGAAATT 120
   GTAAATAACA TCAGGCGTTT GATGGATGGA GAAGAACCTT TGCCAATGCT TCCAAGTTAC 180
   AAGAACTTCA AGGGTACTAT TGAAGAACTG GCTCCAAATC AATATGTGAT TAGTGGTGAA 240
   GTAGCTATTC TTAATTCTAC AACCATTGAA ATCTCAGAGC TTCCCGTCAG AACATGGACC 300
   CAGACATACA AAGAACAAGT TCTAGAACCC ATGTTGAATG G 341
(2) INFORMATION FOR SEQ ID NO:11:
   (i) SEQUENCE CHARACTERISTICS:
      (A) LENGTH: 220 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: YES
   (xi) SEQUENCE DESCRIPTION: SEQ ID NO:11:
   AATTCAAAGC TGGATCCCTT TTAGTTCCTT TTGGGGCAGC CCTTTTTTTG GCACCGGTAG    60
   TGGAGGTGGA AGACTGACTT TTTGCTGCTG TCTTCTTCAC TGTCACATTC TTTTTAGGAA   120
   CTGGGTTTGT AATTTCAGTT TCATCTGGGA AATGTGTAGC AGGAGGGCTT GAAGACAGTG   180
   GTACACTGCC CTTAACATCA TCAGCTTCAA GGTCTGACAC                         220
(2) INFORMATION FOR SEQ ID NO:12:
   (i) SEQUENCE CHARACTERISTICS:
      (A) LENGTH: 170 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: YES
   (xi) SEQUENCE DESCRIPTION: SEQ ID NO:12:
   GTGTTGAGCC TGAATGGTAC ATTCCTATTA TTCCCATGGT GCTGATAAAT GGTGCTGAAG    60
   GAATCGGTAC TGGGTGGTCC TGCAAAATCC CCAACTTTGA TGTGCGTGAA ATTGTAAATA   120
   ACATCAGGCG TTTGATGGAT GGAGAAGAAC CTTTGCCAAT GCTTCCAAGT              170
(2) INFORMATION FOR SEQ ID NO:13:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (xi) SEQUENCE DESCRIPTION: SEQ ID NO:13:
   AATCATCGAT GGATGGATGG 20
(2) INFORMATION FOR SEQ ID NO:14:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: YES
   (xi) SEQUENCE DESCRIPTION: SEQ ID NO:14:
   CCATCCATCCATCGATGATTAAA 23
(2) INFORMATION FOR SEQ ID NO:15;
   (i) SEQUENCE CHARACTERISTICS:
      (A) LENGTH: 327 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: YES
   (xi) SEQUENCE DESCRIPTION: SEQ ID NO:15:
   CTTGATCCCT TCTGGTTGAT GCCAGAAGCT CTTCCTGATC CAGCATTTGT ATCTTCAATT 60
   TCTCTACCAA TTGGCTTTGT TGGTTAATCT CTTCATCCTT GTCATCAAGT TGTTTATACA 120
   ATTTAGCAAG TTCTTCTTCA CACTTTCTTC TTTCAGCATC GGTAAAACTA CCAGCCATTC 180
   CGACTGCAGC AGCTGGTTTA TCACTGGTAA TAGCAATATC TTTATCCGCT GTGAAGGCTT 240
   CCAAATTAGC TTTCTCTTTG TCAAACTGCT CATCAATAGG CACTGTCTCC CCGTTACGCC 300
   AACGGTTTAG CTCGTTTTCC AGCCACT 327
(2) INFORMATION FOR SEQ ID NO:16:
   (i) SEQUENCE CHARACTERISTICS:
      (A) LENGTH: 250 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (xi) SEQUENCE DESCRIPTION: SEQ ID NO:16:
   CCGACCGGGA GCGGGAGAAG GAGCGGGAGC GGGAGCAGCG GGAGAAGGAG CGGGAGAAGG     60
   AGCTGGAGCG CGACGGGAGA AGGAACGGGA GCGCGAGCTG GAGCGGCAGC GGGAGCAGCG    120
   GGCGAGGGAG AAGGAGCTGC TGGCTGCCAA GGCCTTAGAG CCCACCACCT TCCTGCCTGT    180
   GGCCGAGCTG CACGGACTCC GAGGTCACAG CACGGAGGAG CGGCCCAAGC CCTCGGAGCA    240
   GCTGACCCCA                                                           250
(2) INFORMATION FOR SEQ ID NO:17:
   (i) SEQUENCE CHARACTERISTICS:
      (A) LENGTH: 208 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (xi) SEQUENCE DESCRIPTION: SEQ ID NO:17:
   CTCAGAGGTG ATCCTCTCGG AGTCGAGCTC AGGAGAAGGA GTCCCCTTCT TTGAGACTTG     60
   GATGCAGACC TGCATGTCCG AGGAGGGCAA GATTTTGAAC CCTGACCATC CCTGCTTCCG    120
   CCCTGACTCC ACCGAAGTCG AGTCCTTGGT GGCCCTGCTC AACAACTCTT CAGAGATGAA    180
   GCTAGTACAG ATGAAGTAGC ACGAGGCC                                       208
(2) INFORMATION FOR SEQ ID NO:18:
   (i) SEQUENCE CHARACTERISTICS:
      (A) LENGTH: 2389 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: YES
   (xi) SEQUENCE DESCRIPTION: SEQ ID NO:18:
   CGACAAACAT CATCTGGGAA GACCCACACG ATGGAGGGTA AACTTCATGA TCCAGAAGGC     60
   ATGGGAATTA TTCCAAGAAT AGTGCAAGAT ATTTTTAATT ATATTTACTC CATGGATGAA    120
   AATTTGGAAT TTCATATTAA GGTTTCATAT TTTGAAATAT ATTTGGATAA GATAAGGGAC    180
   TTGTTAGATG TTTCAAAGAC TAACCTTTCA GTCCATGAAG ACAAAAACCG TGTTCCCTAT    240
   GTAAAGGGGT GCACAGAACG TTTCGTGTGT AGTCCAGATG AAGTCATGGA TACCATAGAT    300
   GAAGGGAAAT CCAACAGAGA TGTCGCAGTT ACAAATATGA ATGAACATAG CTCTAGGAGC    360
   CACAGCATAT TTCTTATTAA TGTAAAACAA GAGAATACAC AAACGGAACA GAAACTCAGT    420
   GGAAAGCTTT ATCTGGTTGA TTTAGCTGGC AGTGAGAAGG TTAGTAAGAC TGGGGCTGAA    480
   GGTGCTGTGC TGGATGAAGC TAAGAACATC AAGAAGTCAC TTTCTGCACT TGGAAATGTC    540
   ATTTCTGCTT TGGCAGAGGG CAGTACCTAT GTTCCTTATC GAGATAGTAA AATGACCAGA    600
   ATTCTTCAAG ATTCATTAGG TGGCAACTGT AGGACCACTA TTGTCATATG CTGCTCTCCA    660
   TCATCATACA ATGAGTCTGA GACAAAGTCA ACACTCCTCT TTGGTCAAAG GGCCAAAACA    720
   ATTAAGAACA CAGTCTGTGT CAATGTAGAG TTAACTGCAG AGCAGTGGAA AAAGAAGTAT    780
   GAAAAAGAAA AGGAAAAAAA TAAGACTCTA CGGAACACTA TTCAGTGGCT GGAAAACGAG    840
   CTAAACCGTT GGCGTAACGG GGAGACAGTG CCTATTGATG AGCAGTTTGA CAAAGAGAAA    900
   GCTAATTTGG AAGCCTTCAC AGCGGATAAA GATACTGCTA TTACCAGTGA TAAACCAGCT    960
   GCTGCAGTCG GAATGGCTGG TAGTTTTACC GATGCTGAAA GAAGAAAGTG TGAAGAAGAA   1020
   CTTGCTAAAT TGTATAAACA GCTTGATGAC AAGGATGAAG AGATTAACCA ACAAAGCCAA   1080
   TTGGTAGAGA AATTGAAGAC ACAAATGCTG GATCAGGAAG AGCTTCTGGC ATCAACCAGA   1140
   AGGGATCAAG ATAATATGCA AGCTGAACTG AATCGCCTCC AAGCAGAAAA TGATGCTTCT   1200
   AAAGAAGAAG TCAAAGAAGT TTTACAGGCC TTAGAGGAAC TGGCTGTTAA TTATGATCAG   1260
   AAGTCTCAGG AAGTTGAAGA CAAAACAAAG GAATATGAAT TGCTTAGTGA TGAATTGAAT    1320
   CAAAAATCTG CAACTTTAGC AAGTATTGAT GCTGAGCTTC AGAAGCTGAA GGAAATGACC    1380
   AACCACCAGA AGAAACGAGC AGCTGAAATG ATGGCATCAT TATTAAAAGA CCTTGCAGAA    1440
   ATAGGAATTG CTGTGGGGAA TAACGATGTG AAGCAACCAG AAGGAACTGG TATGATAGAT    1500
   GAAGAGTTTA CTGTTGCAAG ACTCTACATT AGCAAAATGA AATCAGAAGT AAAGACCATG    1560
   GTGAAACGCT GCAAACAGCT AGAAAGCACG CAGACTGAGA GCAACAAAAA AATGGAAGAA    1620
   AATGAGAAAG AGTTAGCAGC ATGCCAGCTT CGGATCTCCC AACATGAAGC CAAAATCAAG    1680
   TCACTGACTG AGTACCTTCA GAATGTAGAA CAAAAGAAGA GGCAGCTGGA GGAATCTGTT    1740
   GATTCCCTTG GTGAGGAGCT AGTCCAACTC CGAGCACAAG AGAAAGTCCA TGAAATGGAA    1800
   AAAGAGCACT TGAACAAGGT TCAGACTGCA AATGAAGTCA AGCAAGCTGT TGAGCAGCAG    1860
   ATCCAGAGTC ACAGAGAAAC CCACCAAAAA CAAATCAGTA GCTTGCGAGA TGAAGTTGAG    1920
   GCAAAGGAAA AGCTAATCAC TGACCTCCAA GACCAAAACC AGAAGATGGT GTTGGAGCAG    1980
   GAACGGCTAA GGGTGGAGCA TGAGAGGCTG AAGGCTACAG ACCAAGAGAA GAGCAGGAAG    2040
   CTGCATGAGC TCACGGTTAT GCAAGACAGA CGAGAACAAG CAAGACAAGA CTTGAAGGGT    2100
   TTGGAGGAGA CCGTGGCAAA AGAACTTCAG ACTTTACACA ACCTGCGTAA GCTCTTTGTT    2160
   CAGGACTTGG CTACCAGGGT GAAAAAGAGG CCGAGGTCGA CTCTGACGAC ACTGGCGGCA    2220
   GTGCTGCACA GAAGCAGAAA ATCTCCTTCC TTGAAAACAA CCTTGAACAG CTCACCAAAG    2280
   TGCACAAGCA GTTGGTACGT GATAATGCAG ATCTTCGCTG TGAGCTTCCT AAGTTAGAGA    2340
   AACGGCTTAG AGCTACTGCA GAAAGAGTGA AAGCTTTGGA GTCAGCCCG               2389
(2) INFORMATION FOR SEQ ID NO:19:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: YES
   (xi) SEQUENCE DESCRIPTION: SEQ ID NO:19:
   AGTGGCTGGA AAACGAGCTA 20
(2) INFORMATION FOR SEQ ID NO:20:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: YES
   (xi) SEQUENCE DESCRIPTION: SEQ ID NO:20:
   CTTGATCCCT TCTGGTTGAT 20
(2) INFORMATION FOR SEQ ID NO:21:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: YES
   (xi) SEQUENCE DESCRIPTION: SEQ ID NO:21:
   AGTGGCTTGA AAATGAGCTC 20
(2) INFORMATION FOR SEQ ID NO:22:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: YES
   (xi) SEQUENCE DESCRIPTION: SEQ ID NO:22:
   CTTGATCCCT TCTGGTAGAT G 21